# EUROPEAN PATENT SPECIFICATION

(11) **EP 4 100 044 B1**
(45) Date of publication and mention of the grant of the patent: **01.04.2026**
(21) Application number: 21750969.4
(22) Date of filing: 03.02.2021
(51) Int. Cl.: A61L 26/00, A61L 27/18, A61L 27/50, A61L 27/54, A61K 9/00, A61K 38/17, A61K 47/10, A61K 47/18, A61K 47/26, A61P 19/08, A61P 19/00, A61P 17/02, A61K 47/20, A61K 47/22, A61K 47/36, A61P 1/02, A61P 19/04

(54) **COMPOSITIONS COMPRISING AMELOGENIN AND USES THEREOF**
ZUSAMMENSETZUNGEN MIT AMELOGENIN UND VERWENDUNGEN DAVON
COMPOSITIONS COMPRENANT DE L'AMÉLOGÉNINE ET LEURS UTILISATIONS

(30) Priority: 05.02.2020 IL 27247120
(43) Date of publication of application: 14.12.2022
(73) Proprietor: Prudentix Ltd., Lod 7110401 (IL)
(72) Inventor: SACKS, Hagit, 7173187 Modi'in (IL); KATSMAN, Svetlana, 7048516 Gedera (IL); STERN, Meir, 7622358 Rehovot (IL); LIAPIS, Igal, Vaughan, Ontario L4J6N4 (CA); ROSENBAUM, Danny, 4423564 Kfar Saba (IL); SEGAL, David Dadi, 6436805 Tel Aviv (IL)
(74) Representative: De Clercq & Partners
(86) International application number: PCT/IL2021/050121
(87) International publication number: WO 2021/156857

(56) References cited:
- WO-A1-2018/158764
- WO-A2-2006/064381
- WO-A2-2011/051457
- WO-A2-2011/051457
- GESTRELIUS, STINA ET AL.: "Formulation of enamel matrix derivative for surface coating: kinetics and cell colonization", JOURNAL OF CLINICAL PERIODONTOLOGY, vol. 24.9, 1997, pages 678 - 684, XP002116327, DOI: 10.1111/ j.1600- 05IX.1997 .tb00249.x

## Description

### TECHNICAL FIELD

The present invention relates to pharmaceutical compositions comprising amelogenin, which are useful for promoting periodontal or orthopedic soft or hard tissue regeneration, wound closure, and skin regeneration and rejuvenation.

### BACKGROUND ART

Amelogenin, X-Linked (AmelX), is the principal protein in developing dental enamel, and belongs to a family of extracellular matrix proteins, which regulate the size, shape, and the oriented and elongated growth of the enamel mineral crystal. The protein is hydrophobic and has an isoelectric point of 6.6-6.8. Therefore, it is typically present in a monomeric form at low pH and at a low temperature, e.g., at about 4-20°C, and tends to self-assemble into nano-sized spherical aggregates having diameters of 10-100 nm ("nano-spheres"), which then form three-dimensional networks at body temperature and physiological pH. Such changes in conformation and solubility could have a direct impact on protein precipitation during clinical applications. Said self-assembly process involves hydrophobic interactions, and it is sensitive not only to pH but also to variations in temperature.

In order to be therapeutically effective, amelogenin, when administered as a therapeutic agent to a subject, should be in its native conformation. However, during manufacture (i.e., extraction a natural protein from a mammal, or producing as a recombinant protein; final composition preparation, packing) storage and delivery, the protein may undergo denaturation and/or precipitate in the form of irreversible aggregates.

In this respect, it should be noted that amelogenins show an overall high level of sequence homology, more particularly about 80%, among all higher vertebrates studied. In fact, the sequences of human and porcine amelogenin gene transcripts have identity of about 96%. Therefore, both enamel matrix proteins (EMP) and enamel matrix derivatives (EMD) that are of porcine origin are considered "identical" when administered to human, and therefore do not cause any allergic response or other unfavorable reaction.

Emdogain^{®} is a therapeutic gel currently used for regenerative treatment of periodontal disease, to promote the regrowth of hard and soft tissues lost due to periodontal disease. The active component of Emdogain^{®} is EMD that is obtained from a porcine dental tissue extract, containing different extra-cellular proteins including 90% amelogenin. This therapeutic gel contains a relatively high concentration, i.e., 30 mg/ml, of EMD, as well as propylene glycol alginate (PGA) acting as a carrier. However, PGA degrades over time and reduces the pH of the product. In addition, the isolated EMD is heated for 3 hours at about 80°C in order to inactivate residual proteases, and this necessary heat-treatment has recently been found to be a key factor in bringing on an irreversible degradation and denaturation of the EMD itself and in turn contribute to a shorter shelf-life and/or less activity of the formulation.

Recent data obtained from a series of *in vitro* studies revealed that the use of EMD without its PGA carrier markedly improved protein adsorption when compared to conventional Emdogain (Miron *et al.,* 2015). A further advantage of using EMD without its PGA carrier is related to its fluid consistency and insufficient surface coating and penetration of the EMD. More specifically, due to insufficient surface coating and penetration of the Emdogain^{®} gel, its use in dental defects with complicated anatomy, e.g., non-contained-type defects, might be limited (Mellonig 1999, Lekovic *et al.,* 2000, Cochran *et al.,* 2003, Shirakata *et al.,* 2007, Sculean *et al.,* 2007).

Currently, there is a need for stable and biologically potent amelogenin-based products. Such products should be sterile, stable, reproducible in large quantities, and easily injectable, and should preferably enable releasing the amelogenin, at the desired site, in a sustained manner.

WO 2018/158764 discloses a liquid composition comprising a non-biodegradable thermosensitive polyalkylene oxide block copolymer, e.g., a poloxamer, a low molecular weight hyaluronic acid, and optionally a therapeutic agent, which solidifies into a viscous gel upon warming to body temperature and then releases said hyaluronic acid and said therapeutic agent, when present, in a sustained release manner.

WO 2011/051457 discloses a pharmaceutical composition consisting of purified EMD proteins formulated in a suitable pharmaceutical carrier intended to be used for promoting and/or inducing regeneration of hard tissue, tissue mineralization, bone growth and/or bone regrowth, regeneration of dentin, cementogenesis, and/or binding between parts of living mineralized tissue, for bonding of a piece of living mineralized tissue to a bonding site on a piece of other living tissue, for endorsing binding between hard tissues, and/or for filling a mineralized wound cavity and/or tissue defect following from a procedure and/or trauma.

### SUMMARY OF INVENTION

Disclosed herein are two medical compositions containing amelogenin as the active agent, wherein one of the compositions (referred to herein as a ***"poloxamer copolymer-based composition***") is in the form of a liquid under refrigerated conditions, i.e., at a temperature of about 2-8°C, and upon contacting with a body tissue thereby warming to body temperature, solidifies into a viscous gel; and the other composition, which is disclosed but not claimed, (referred to herein as a *"**hyaluronic acid-based composition***) is in the form of a gel (both under refrigerated conditions and at ambient temperature).

As shown herein, the poloxamer copolymer-based composition of the invention, which can be manufactured aseptically, enables maintaining the active agent in acidic pH, preventing premature precipitation, and preserving amelogenin stability and functional integrity; and may be administered in various potent concentrations. This liquid composition has optimal viscosity for clinical application; however, upon administration to a body tissue and consequently warming to body temperature, it solidifies into a viscous gel that adheres to the treated site, and enables easy, precise and sustained release of the active agent.

As further shown, the hyaluronic acid-based composition is a mucoadhesive gel combining the anti-inflammatory and mucoadhesive properties of the hyaluronic acid with the regenerative properties of amelogenin. This composition, which can be manufactured aseptically with flexible doses of the active agent, and stored intact for a long period, enables preventing premature precipitation, maintaining amelogenin stability and functional integrity, and upon administration to a treated site of a subject - releasing the active agent in a sustained manner.

In one aspect, the present invention thus provides a liquid medical composition (a "***poloxamer copolymer-based composition***") comprising a non-biodegradable thermosensitive pharmaceutically acceptable poloxamer copolymer in an amount of 18-30% by weight; amelogenin in an amount of 0.005-3% by weight; a disaccharide in an amount of 0.05-5% by weight; an amino acid selected from alanine, glycine, isoleucine, leucine, proline, valine, and a mixture thereof in an amount of 0.05-5% by weight; and an antioxidant which is an o-quinone scavenger selected from ascorbic acid or a salt thereof, ascorbic acid derivative such as ascorbic acid-6-palmitate, methionine, N-acethylmethionine, cysteine, N-acetyl cysteine (NAC), and gluthatione (GSH), in an amount of 0.01-2% by weight, wherein said composition has a pH of 3.8 to 5.5, preferably a pH of 3.8 to 5.0, and a viscosity of 50-2000, preferably 100-1000, *centipoise* (cP; also referred to as millipascal seconds, mPa·sec) at a temperature of about 2-8°C, preferably about 4-6°C, when measured with Rheometer MCR 72 (Anton Paar GmbH) under shear rate of 1000 sec⁻¹; and upon warming to about 37°C, said composition solidifies into a gel having a viscosity not less than 10000 cP when measured using the same device at a shear rate of 1 sec⁻¹.

In certain embodiments, the poloxamer copolymer is poloxamer 407, poloxamer 188, poloxamer 124, poloxamer 237, poloxamer 338, or a mixture thereof. In particular such embodiments, said poloxamer copolymer is poloxamer 407.

In certain embodiments, the poloxamer copolymer is present in said composition in an amount of 21% to 29%, 23% to 28%, or 25% to 27%, by weight.

In certain embodiments, the amelogenin is a natural amelogenin or a recombinant amelogenin. In particular such embodiments, said amelogenin is present in said composition in an amount of 0.01% to 1% by weight.

In certain embodiments, the present invention provides a composition as defined above, wherein: (i) said disaccharide is sucrose, trehalose, lactose, maltose, cellobiose, gentiobiose, or a mixture thereof; (ii) said amino acid is glycine; or (iii) said antioxidant is L-methionine, N-acethylmethionine, L-cysteine, N-acetyl cysteine (NAC), glutathione (GSH), ascorbic acid, an ascorbate such as sodium ascorbate, an ascorbic acid derivative such as ascorbic acid-6-palmitate, or a mixture thereof. In particular such embodiments, (i) said disaccharide is present in said composition in an amount of 0.1% to 2.5% by weight; (ii) said disaccharide is present in said composition in an amount of 0.1% to 2.5% by weight; or (iii) said antioxidant is present in said composition in an amount of 0.05% to 1% by weight.

In certain embodiments, said poloxamer copolymer is poloxamer 407; said amelogenin is a recombinant amelogenin; said disaccharide is trehalose; said amino acid is glycine; and said antioxidant is methionine. In particular embodiments, said poloxamer copolymer is present in said composition in an amount of 21% to 29% by weight; said amelogenin is present in said composition in an amount of 0.01% to 1% by weight; said disaccharide is present in said composition in an amount of 0.1% to 2.5% by weight; said amino acid is present in said composition in an amount of 0.1% to 1% by weight; said antioxidant is present in said composition in an amount of 0.05% to 1% by weight; and said composition has a pH of 4.0 to 5.0. In more particular such embodiments, said poloxamer copolymer is present in said composition in an amount of 25% to 27% by weight; said amelogenin is present in said composition in an amount of 0.02% to 0.5% by weight; said disaccharide is present in said composition in an amount of 0.2% to 1% by weight; said amino acid is present in said composition in an amount of 0.2% to 0.5% by weight; and said antioxidant is present in said composition in an amount of 0.1% to 0.5% by weight.

In certain embodiments, the poloxamer copolymer-based composition according to any one of the embodiments above, further comprises a mucoadhesive agent such as a low molecular weight hyaluronic acid (HA), propylene glycol alginate (PGA), xanthan gum, gum acacia, a carbomer (polyacrylic acid), chitosan, human serum albumin, gellun gum, guar gum, carrageenan, and a cellulose derivative such as carboxymethyl cellulose and hydroxypropyl methylcellulose; and/or a buffering agent such as acetic acid-, citric acid-, tartaric acid-, lactic acid-, hydrochloric acid-, or hydrogenphosphoric acid-based buffer, diethylamine-based buffer, Britton-Robinson buffer, and Carmody buffer.

Also disclosed herein is a medical composition in a form of a gel (a "***hyaluronic acid-based composition***)*,* said composition comprising a high molecular weight hyaluronic acid (HA; also called hyaluronan or hyaluronate) or a salt thereof in an amount of 0.5-10%; amelogenin in an amount of 0.005-3% by weight; a sugar in an amount of 0.05-5% by weight; an amino acid in an amount of 0.05-5% by weight; and an antioxidant in an amount of 0.01-2% by weight, wherein said composition has a pH of 3.8 to 5.5, preferably a pH of 3.8 to 5.0. This composition, as well as the methods of use and kit disclosed herein and referring thereto, are not encompassed by the wording of the claims and are disclosed for illustration purposes only.

The medical compositions disclosed herein are useful in treatment of medical conditions wherein application/administration of amelogenin is beneficial. For example, such compositions are useful for promoting periodontal soft or hard tissue regeneration, e.g., regeneration of a tissue lost due to periodontal disease such as periodontitis or gingival recession, or periodontal trauma such as oral or periodontal wound. In other embodiments, said compositions are useful for promoting orthopedic soft or hard tissue regeneration, e.g., regeneration of bone, cartilage, ligaments, or tendons. In further embodiments, these compositions are useful for promoting wound closure, i.e., wound healing, e.g., for assisting in dermal repair and regeneration, or reducing scarring tissue. In still other embodiments, said compositions are useful in promoting skin regeneration and rejuvenation, i.e., by induction/stimulation of collagen production and proliferation, or maintenance of skin moisture, for improved skin appearance and wrinkle mitigation.

In another aspect, the present invention relates to a medical composition as defined above, i.e., a poloxamer copolymer-based composition, for use in promoting (i) periodontal or orthopedic soft or hard tissue regeneration; (ii) wound closure, i.e., wound healing; or (iii) skin regeneration and rejuvenation.

In certain embodiments, said orthopedic soft tissue is cartilage, ligaments, or tendons; said orthopedic hard tissue is bone; or said periodontal soft or hard tissue is a tissue lost due to periodontal disease such as periodontitis or gingival recession, or periodontal trauma such as oral or periodontal wound. In particular such embodiments, said composition is for (i) treatment of intrabony defects, furcation defects, or recession defects; (ii) treatment of articular cartilage defects, cartilage or osteochondral defects, or mild to moderate osteoarthritis including multiple defects, thereby repairing joint surface lesions, or cartilage focal lesions; (iii) treatment of anterior cruciate ligament injury, Grade II and III acute lateral ankle ligament injury, or scapholunate interosseous ligament injury / scapholunate ligament tear / torn wrist ligaments; or (iv) treatment of tendon injury, lateral epicondylitis, flexor tendon injury, lesions of the fingers' flexor tendons, injured tendon of the ankle, non-ruptured tendon injuries, or tendinopathy.

In other embodiments, said promoting wound closure is assisting in dermal repair and regeneration, or reducing scarring tissue.

In yet another aspect, the present invention relates to a kit comprising a poloxamer copolymer-based composition as defined above, and a delivery mean, e.g., a syringe or an applicator, for administering or applying said composition to a site in a subject in need of periodontal or orthopedic soft or hard tissue regeneration, wound closure, or skin regeneration and rejuvenation.

### DETAILED DESCRIPTION

In one aspect, the present invention provides a poloxamer copolymer-based composition as defined above, i.e., a liquid medical composition comprising a non-biodegradable thermosensitive pharmaceutically acceptable poloxamer copolymer in an amount of 18-30% by weight; amelogenin in an amount of 0.005-3% by weight; a disccharide in an amount of 0.05-5% by weight; an amino acid selected from alanine, glycine, isoleucine, leucine, proline, valine, and a mixture thereof in an amount of 0.05-5% by weight; and an antioxidant which is an o-quinone scavenger selected from ascorbic acid or a salt thereof, ascorbic acid derivative such as ascorbic acid-6-palmitate, methionine, N-acethylmethionine, cysteine, NAC, and GSH, in an amount of 0.01-2% by weight, wherein said composition has a pH of 3.8 to 5.5 and a viscosity of 50-2000 mPa·sec at a temperature of about 2-8°C, when measured with Rheometer MCR 72 under shear rate of 1000 sec⁻¹; and upon warming to about 37°C, said composition solidifies into a gel having a viscosity of at least 10000 mPa·sec when measured with Rheometer MCR 72 under shear rate of 1 sec⁻¹.

Poloxamers have been widely used in the biomedical field due to their ability to undergo phase reverse thermal gelation. Their self-assembling process occurs through micellization, which is characterized by their critical micellization concentration and critical micellization temperature. These parameters, which depend on the specific poloxamer used and its concentration, as well as on the excipients added to the poloxamer and the concentration thereof, can be tailored to obtain materials with final properties suitable for a wide range of applications. Poloxamer gels are "generally regarded as safe" (GRAS) excipients and have been widely investigated and used for delivery of active agents. One of the drawbacks associated with poloxamer gels for delivery applications is short residence times due to lack of adhesiveness. Blending of poloxamers with mucoadhesive polymers that are capable of forming entanglements or non-covalent bonds with the mucus covering epithelial tissues is therefore one of the approaches to improve adhesiveness and residence time.

The term "poloxamer copolymer" as used herein denotes a polyethoxy/ polypropoxy block copolymer, i.e., a nonionic triblock copolymer composed of a central hydrophobic chain of polyoxypropylene (poly(propylene oxide)) flanked by two hydrophilic chains of polyoxyethylene (poly(ethylene oxide)). Particular examples of such poloxamers include, without being limited to, poloxamer 407, poloxamer 188, poloxamer 124, poloxamer 237, poloxamer 338, or a mixture thereof. In particular embodiments, the poloxamer comprised within the poloxamer copolymer-based composition is poloxamer 407.

The present medical composition as claimed herein does not require the use of poloxamer copolymers having a specific level of purity, and thus polymers of any grade of purity may be employed. Moreover, the medical composition disclosed herein may contain more than one thermosensitive pharmaceutically acceptable polymer. It is, however, preferable to employ polymers having a high degree of purity, and especially a defined (i.e., specifiable) composition, since the use of such polymers increases the degree with which the release of the therapeutic agent, i.e., amelogenin, may be controlled.

In certain embodiments, the non-biodegradable thermosensitive pharmaceutically acceptable poloxamer copolymer composing the poloxamer copolymer-based composition, according to any one of the embodiments above, is present in said composition in an amount of from about 21% to about 29%, about 23% to about 28%, about 25% to about 27%, e.g., in an amount of about 25%, about 25.25%, about 25.5%, about 25.75%, about 26%, about 26.25%, about 26.5%, about 26.75%, or about 27%, by weight.

In certain embodiments, the amelogenin comprised within the poloxamer copolymer-based composition is a natural amelogenin, i.e., a human amelogenin (including a recombinant human amelogenin), or an amelogenin derived from a non-human animal such as a pig. Recombinant human amelogenin may be produced utilizing any suitable expression and purification system and method as described in the literature. For instance, Bonde and Bulow (2012) disclose a method for purification of a recombinant human amelogenin expressed in *Escherichia coli,* based on the solubility properties of amelogenin. Said method combines cell lysis with recovery/purification of the protein and enables obtaining >95% pure amelogenin in one step using intact harvested cells as starting material. A recombinant human amelogenin was also expressed and characterized in yeast expression system, more specifically in *Pichia pastoris* (Cheng *et al.,* 2012); and in a eukaryotic baculovirus system. Specifically, the recombinant protein was expressed in *Spodoptera frugiperda* (Sf9) insect cells and the yield of purified his-tagged human amelogenin (rHAM⁺) was up to 10 mg/L culture (Taylor *et al.,* 2006). In particular embodiments, the amelogenin comprised within said composition is the recombinant human amelogenin rAmelX (produced from *E. coli*; Merck-Sigma), which has a molecular weight of 19935.87 g/mol; extinction coefficient of 26030 M⁻¹cm⁻¹; isoelectric point of 6.6-6.8; and the following sequence: NH₂-MPLPPHPGHP GYINFSYEVL TPLKWYQSIR PPYPSYGYEP MGGWLHHQII PVLSQQHPPT HTLQPHHHIP VVPAQQPVIP QQPMMPVPGQ HSMTPIQHHQ PNLPPPAQQP YQPQPVQPQP HQPMQPQPPV HPMQPLPPQP PLPPMFPMQP LPPMLPDLTL EAWPSTDKTK REEVD-COOH (SEQ ID No. 1). In particular such embodiments, the amelogenin is present in said composition in an amount of about 0.01% to about 1%, 1.5%, 2%, or 2.5%, about 0.025% to about 0.75%, about 0.05% to about 0.5%, about 0.075% to about 0.25%, or about 0.1% to about 0.2%, by weight.

Many proteins are structurally unstable in solutions and are susceptible to conformational changes due to various stresses encountered during purification, processing, and storage. Therefore, a wide variety of protein stabilizing agents have been used for enhancing the stability of both pharmaceutical and reagent proteins. According to the literature, such stabilizing agents are capable of stabilizing the structure of particular native proteins at moderate (0.1 M) to high concentrations (1 M). Particular stabilizing agents often used so as to protect proteins from aggregation include sugars and polyols (Ohtake *et al.,* 2011).

The term "sugar" as used herein with respect to the medical compositions as claimed herein refers to a soluble carbohydrate (a molecule containing carbon, hydrogen and oxygen atoms, which can be cyclic or linear, saturated or unsaturated, and substituted or unsubstituted), and include *inter alia* a monosaccharide such as glucose, fructose, galactose, D-mannose, and sorbose; a sugar alcohol, i.e., polyol, which is an organic compound typically derived from a monosaccharide and containing one hydroxyl group attached to each carbon atom, such as mannitol and sorbitol; a disaccharide, i.e., a molecule composed of two monosaccharides linked via a glycosidic bond, such as sucrose (β-D-fructofuranosyl α-D-glucopyranoside) and trehalose ((2*R*,3*S*,4*S*,5*R*,6*R*)-2-(hydroxymethyl)-6-[(2*R*,3*R*,4*S*,5*S*,6*R*)-3,4, 5-trihydroxy-6-(hydroxymethyl)oxan-2-yl] oxyoxane-3,4,5-triol); lactose (B-D-galactopyranosyl-(1-4)-D-glucose), maltose ((3*R,*4*R,*5*S,*6*R*)*-*6-(hydroxymethyl)-5-{[(2*R*,3*R*,4*S*,5*S*,6*R*)-3,4,5-trihydroxy-6-(hydroxymethyl)oxan-2-yl]oxy}oxane-2,3,4-triol); cellobiose ( 4-O-(3-D-glucopyranosyl-(3-D-glucopyranose); and gentiobiose (6-O-β-D-glucopyranosyl-D-glucose); an oligosaccharide; or a polysaccharide such as mannotriose, raffinose, melezitose, a maltodextrin, i.e., a chain consisting of a variable number (e.g., 3-17) of D-glucose units linked with α(1→4)glycosidic bonds, or a dextran, i.e., a complex branched glucan (polysaccharide of D-glucose monomers linked by glycosidic bonds); or a cyclodextrin, particularly a cyclodextrin having six, seven or eight glucopyranose units, i.e., α-, β- or γ-cyclodextrin, respectively.

Particular examples of cyclodextrins include hydroxyalkyl-cyclodextrins, more particularly hydroxyalkyl-α-, hydroxyalkyl-β- or hydroxyalkyl-γ-cyclodextrins, but preferably hydroxyalkyl-β-cyclodextrins such as hydroxyethyl-β-cyclodextrin, hydroxypropyl-β-cyclodextrin (e.g., 2-hydroxypropyl-(3-cyclodextrin), dihydroxypropyl-β-cyclodextrin, or hydroxybutyl-β-cyclodextrin. The term "hydroxyalkyl" as used herein refers to any hydroxyl derivative of a C₁-C₄ alkyl, i.e., a straight or branched saturated hydrocarbon radical having 1-4 carbon atoms such as methyl, ethyl, *n*-propyl, isopropyl, *n*-butyl, sec-butyl, isobutyl and tert-butyl.

Other examples of cyclodextrins include sulphoalkylether-cyclodextrins, more particularly sulphoalkylether-α-, sulphoalkylether-β- or sulphoalkylether-γ-cyclodextrin, preferably sulphoalkylether-β-cyclodextrin such as sulphoethylether-β-cyclodextrin, sulphopropylether-β-cyclodextrin, sulphobutylether-β-cyclodextrin, or sulphopentylether-β-cyclodextrin. The term "sulphoalkylether" as used herein refers to a group of the general formula -O-(C₁-C₄)alkylene-SO₃H, wherein (C₁-C₄)alkylene typically means a divalent straight or branched hydrocarbon radical having 1-4 carbon atoms such as methylene, ethylene, propylene, butylene and 2-methylpropylene.

The degree of substitution may have an effect on the binding of guests to the hydroxyalkyl- or sulphoalkylether-cyclodextrin molecule, wherein at low degrees of substitution, binding is very similar to that of the unmodified cyclodextrin, while increasing substitution can lead to weakened binding due to steric hindreance. The effect on the binding of guests to the host molecule is dependent upon the particular guest and it is also possible to obtain increased binding due to an increase in surface area to which the guest can bind. Still, with most guests, these differences in binding with degree of substitution are small if detectable.

The poloxamer copolymer-based composition comprises at least one disaccharide as one of the agents aimed at stabilizing the amelogenin. In certain embodiments, said disaccharide is lactose, maltose, cellobiose, gentiobiose, a non-reducing disaccharide such as sucrose and trehalose, or a mixture thereof. Preferred are compositions wherein said disaccharide is trehalose. In particular such embodiments, the disaccharide is present in said composition in an amount of about 0.1% to about 2.5%, 3%, 3.5%, 4%, or 4.5%, e.g., about 0.25% to about 2.5%, about 0.5% to about 2%, about 0.75% to about 1.5%, or about 1% to about 1.25%, by weight.

Amino acids have been evaluated as potential stabilizing additives due to their broad range of sizes and physicochemical properties. A study exploring the use of amino acids for stabilizing proteins in solution showed an improvement in stability of protein formulation by the addition of amino acids. The results of this study suggest that "the fit" of amino acids into the "free volume holes" of the amorphous protein-based formulations matrix is important in suppressing dynamics on a fast timescale, thereby leading to improved stability. Commonly, amino acids are used in liquid protein formulations as buffers, and so as to stabilize the protein structure and improve stability of the protein formulation (Forney-Stevens *et al.,* 2016).

The term "amino acid" as used herein refers to an organic compound comprising both amine and carboxylic acid functional groups, which may be either a natural or non-natural amino acid and occur in both L and D isomeric forms. The twenty-two amino acids naturally occurring in proteins areaspartic acid (Asp), tyrosine (Tyr), leucine (Leu), tryptophan (Trp), arginine (Arg), valine (Val), glutamic acid (Glu), methionine (Met), phenylalanine (Phe), serine (Ser), alanine (Ala), glutamine (Gln), glycine (Gly), proline (Pro), threonine (Thr), asparagine (Asn), lysine (Lys), histidine (His), isoleucine (Ile), cysteine (Cys), selenocysteine (Sec), and pyrrolysine (Pyl). Non-limiting examples of other amino acids include citrulline (Cit), diaminopropionic acid (Dap), diaminobutyric acid (Dab), ornithine (Orn), aminoadipic acid, β-alanine, 1-naphthylalanine, 3-(1-naphthyl)alanine, 3-(2-naphthyl)alanine, γ-aminobutiric acid (GABA), 3-(aminomethyl) benzoic acid, *p*-ethynyl-phenylalanine, *m*-ethynyl-phenylalanine, *p*-chlorophenylalanine (4ClPhe), *p*-bromophenylalanine, *p*-iodophenylalanine, *p*-acetylphenylalanine, *p-*azidophenylalanine, *p*-propargly-oxy-phenylalanine, indanylglycine (Igl), (benzyl)cysteine, norleucine (Nle), azidonorleucine, 6-ethynyl-tryptophan, 5-ethynyl-tryptophan, 3-(6-chloroindolyl)alanine, 3-(6-bromoindolyl)alanine, 3-(5-bromoindolyl)alanine, azidohomoalanine, α-aminocaprylic acid, O-methyl-L-tyrosine, N-acetylgalactosamine-α-threonine, and N-acetylgalactosamine-α-serine.

The poloxamer copolymer-based composition comprises at least one amino acid selected from alanine, glycine, isoleucine, leucine, proline, and valine, as one of the agents aimed at stabilizing the amelogenin. In certain embodiments, said amino acid is glycine, a mixture thereof, or a mixture of glycine and one or more additional amino acids. Preferred are compositions wherein said amino acid is glycine. In particular such embodiments, the amino acid is present in said composition in an amount of about 0.1% to about 2.5%, 3%, 3.5%, 4%, or 4.5%, e.g., about 0.2%, 0.3% or 0.4% to about 2.5%, about 0.5%, 0.6% or 0.7% to about 2%, about 0.8% to about 1.5%, or about 1% to about 1.25%, by weight.

Antioxidants are compounds that inhibit oxidation, i.e., prevent or delay oxidative degradation of an oxidizable compound, and thus inhibit or limit the formation of free radicals which lead to chain reactions that may damage said oxidizable compound. Antioxidants such as thiols or ascorbic acid terminate these chain reactions.

Examples of antioxidants include, without being limited to, tyrosinase inhibitors such as captopril; o-quinone scavengers such as ascorbic acid or a salt thereof (e.g., Na-ascorbate), ascorbic acid derivatives (e.g., ascorbic acid-6-palmitate), and sulfur containing o-quinone scavengers, e.g., L-methionine (also referred to herein as methionine) (see, e.g., Bittner 2006), N-acethylmethionine, L-cysteine (also referred to herein as cysteine), N-acetyl cysteine (NAC), and gluthatione (GSH); Cu⁺⁺ chelators such as Na₂-EDTA, Na₂-EDTA-Ca, DMSA (succimer), D-penicillamine (DPA), trientine-HCl, dimercaprol, clioquinol, sodium thiosulfate, triethylenetetramine (TETA), tetraethylenepentamine (TEPA), curcumin, neocuproine, tannin, and/or cuprizone; synthetic antioxidants such as butylated hydroxyanisole (BHA), methylated phenols such as tocopherol, and sulfite salts such as sodium hydrogen sulfite or sodium metabisulfite.

In certain embodiments, the antioxidant comprised within the poloxamer copolymer-based composition is an o-quinone scavenger, more specifically is an o-quinone scavenger selected from L-methionine, N-acethylmethionine, L-cysteine, N-acetyl cysteine, glutathione, ascorbic acid, an ascorbate such as sodium ascorbate, an ascorbic acid derivative such as ascorbic acid-6-palmitate, or a mixture thereof. Preferred are compositions wherein said antioxidant is L-methionine. In particular such embodiments, the antioxidant is present in said composition in an amount of about 0.05% to about 1% or 1.5%, e.g., about 0.06% to about 0.8%, about 0.08% to about 0.6%, about 0.1% to about 0.4%, or about 0.5%, by weight.

In certain embodiments, provided is a poloxamer copolymer-based composition as defined in any one of the embodiments above, wherein the non-biodegradable thermosensitive pharmaceutically acceptable poloxamer copolymer is poloxamer 407; said amelogenin is a recombinant amelogenin; said disaccharide is trehalose; said amino acid is glycine; and said antioxidant is methionine. In particular such embodiments, said poloxamer copolymer is present in said composition in an amount of 21% to 29% by weight; said amelogenin is present in said composition in an amount of 0.01% to 1% by weight; said disaccharide is present in said composition in an amount of 0.1% to 2.5% by weight; said amino acid is present in said composition in an amount of 0.1% to 1% by weight; said antioxidant is present in said composition in an amount of 0.05% to 1% by weight; and said composition has a pH of 4.0 to 5.0. More particular such embodiments are those wherein said poloxamer copolymer is present in said composition in an amount of 25% to 27% by weight; said amelogenin is present in said composition in an amount of 0.02% to 0.5% by weight; said disaccharide is present in said composition in an amount of 0.2% to 1% by weight; said amino acid is present in said composition in an amount of 0.2% to 0.5% by weight; and said antioxidant is present in said composition in an amount of 0.1% to 0.5% by weight.

The poloxamer copolymer-based composition as defined in any one of the embodiments above may further comprise a mucoadhesive agent and/or a buffering agent. Examples of mucoadhesive agents include, without limiting, a low molecular weight hyaluronic acid, propylene glycol alginate (PGA), xanthan gum, gum acacia, a carbomer (polyacrylic acid), chitosan, human serum albumin, gellun gum, guar gum, carrageenan, and cellulose derivatives such as carboxymethyl cellulose (CMC) and hydroxypropyl methylcellulose (HPMC); and non-limiting examples of buffering agents include acetic acid-based buffers such as acetic acid-sodium acetate buffer; citric acid-based buffers such as citric acid-disodium hydrogen phosphate buffer, and citric acid-sodium citrate buffer; tartaric acid-, lactic acid-, hydrochloric acid-, and hydrogenphosphoric acid-based buffers; diethylamine-based buffers; Britton-Robinson buffer; and Carmody buffer.

Also disclosed herein, for illustration purposes only, is a hyaluronic acid-based composition as defined above, i.e., a medical composition in a form of a gel, which comprises a high molecular weight hyaluronic acid or a salt thereof in an amount of 0.5-10%; amelogenin in an amount of 0.005-3% by weight; a sugar in an amount of 0.05-5% by weight; an amino acid in an amount of 0.05-5% by weight; and an antioxidant in an amount of 0.01-2% by weight, wherein said composition has a pH of 3.8 to 5.5.

Hyaluronan has important hygroscopic, rheological and viscoelastic properties that fluctuate with changes in temperature, pH, ionic environment, and binding partners. However, these properties are also highly dependent on chain length. Hyaluronan can reach over 10⁷ Da in molecular mass, but also exists in multiple smaller forms, referred to as low-or medium-molecular weight hyaluronan.

Hyaluronan has been found to have tissue healing properties, and it thus could be used as an adjunct to mechanical therapy in the treatment of periodontitis (Sukumar and Drizhal, 2007). As previously shown, high molecular weight hyaluronan are immunosuppressive, antiangiogenic, anti-inflammatory, and possess mucoadhesive properties, and were shown to protect against lymphocyte-mediated cytolysis (McBride and Bard, 1979), suppress septic responses to lipopolysaccharides, maintain immune tolerance, induce production of immunosuppressive macrophages, and reduce expression of inflammatory cytokines. Such hyaluronan was further found to have antiaging and anticancer effects (Morrison *et al.,* 2001; Jiang *et al.,* 2005).

The term "high molecular weight hyaluronic acid" as used herein refers to any hyaluronic acid molecule having a molecular weight of at least 500 kDA, more specifically a molecular weight of about 800 kDA (0.8 MDa) or more. Particular high molecular weight hyaluronic acid composing the hyaluronic acid-based composition has a molecular weight of from about 0.8 MDa to about 6 MDa, about 0.9 MDa to about 4 MDa, or from about 1 MDa to about 2 or 3 MDa, e.g., about 1, 1.1, 1.2, 1.3, 1.4, 1.5, 1.6, 1.7, 1.8, 1.9, or 2 MDa. Said hyaluronic acid may optionally be hydrolyzed or partially hydrolyzed, and may be in its base form or as a pharmaceutically acceptable salt thereof. Examples of hyaluronan salts include, without limiting, alkaline metal or alkaline earth metal salts of hyaluronan, e.g., sodium hyaluronate, potassium hyaluronate, and calcium hyaluronate.

The hyaluronic acid comprised within the hyaluronic acid-based composition may be present in said composition in an amount of about 0.75% to about 8%, about 1% to about 6%, about 1.1% to about 4%, or about 1.2% to about 2%, e.g., about 1.2%, 1.3%, 1.4%, 1.5%, 1.6%, 1.7%, 1.8%, 1.9%, or 2%, by weight.

The amelogenin comprised within the hyaluronic acid-based composition may be a natural amelogenin, i.e., a human amelogenin (including a recombinant human amelogenin), or an amelogenin derived from a non-human animal such as a pig. For example, the amelogenin comprised within said composition may be the recombinant human amelogenin rAmelX referred to above. The amelogenin may be present in said composition in an amount of about 0.01% to about 1%, 1.5%, 2%, or 2.5%, about 0.025% to about 0.75%, about 0.05% to about 0.5%, about 0.075% to about 0.25%, or about 0.1% to about 0.2%, by weight.

The sugar comprised within the hyaluronic acid-based composition may be a polyol such as mannitol, a disaccharide such as trehalose, or a mixture thereof. For example, the sugar may be present in said composition in an amount of about 0.1% to about 2.5%, 3.5%, 4%, or 4.5%, e.g., about 0.25% to about 2.5%, about 0.5% to about 2%, about 0.75% to about 1.5%, or about 1% to about 1.25%, by weight.

The amino acid comprised within the hyaluronic acid-based composition may be glycine, methionine, a mixture thereof, or a mixture of amino acids including glycine and/or methionine. For example, the amino acid may be present in said composition in an amount of about 0.1% to about 2.5%, 3.5%, 4%, or 4.5%, e.g., about 0.2%, 0.3% or 0.4% to about 2.5%, about 0.5%, 0.6% or 0.7% to about 2%, about 0.8% to about 1.5%, or about 1% to about 1.25%, by weight.

The antioxidant comprised within the hyaluronic acid-based composition may be methionine. For example, the antioxidant is present in said composition in an amount of about 0.05% to about 1% or 1.5%, e.g., about 0.06% to about 0.8%, about 0.08% to about 0.6%, about 0.1% to about 0.4% or 0.5%, by weight.

Disclosed herein is a hyaluronic acid-based composition as defined above, wherein the hyaluronic acid has a molecular weight of about 1.2-2 MDa, e.g., about 1.2 MDa, 1.3 MDa, 1.4 MDa, 1.5 MDa, 1.5 MDa, 1.6 MDa, 1.7 MDa, 1.8 MDa, 1.9 MDa, or 2 MDa, and it is optionally hydrolyzed or partially hydrolyzed; said amelogenin is a recombinant amelogenin; said sugar is a mixture of trehalose and mannitol; said amino acid is glycine; and said antioxidant is methionine. For example, said hyaluronic acid is present in said composition in an amount of 0.75% to 8%, by weight; said amelogenin is present in said composition in an amount of 0.01% to 1% by weight; said sugar is present in said composition in an amount of 0.1% to 2.5% by weight; said amino acid is present in said composition in an amount of 0.1% to 1% by weight; said antioxidant is present in said composition in an amount of 0.05% to 1% by weight; and said composition has a pH of 4-5. More particularly, said hyaluronic acid is present in said composition in an amount of about 1.5% to 2% by weight; said amelogenin is present in said composition in an amount of about 0.02% to 0.5% by weight; said sugar is present in said composition in an amount of about 0.2% to 2% by weight; said amino acid is present in said composition in an amount of about 0.2 to 0.5% by weight; and said antioxidant is present in said composition in an amount of about 0.1% to 0.5% by weight.

The hyaluronic acid-based composition disclosed herein may further comprise a surfactant and/or a buffering agent. Examples of surfactants include, without being limited to, a poloxamer such as poloxamer 188 (F-68) and poloxamer 407 (F-127); a polysorbate such as Tween 20, Tween 40, Tween 60 and Tween 80; or a sorbitane such as Span 20; and non-limiting examples of buffering agents include acetic acid-based buffers and citric acid-based buffers, e.g., those listed above.

The term "medical composition" or "pharmaceutical composition" as used herein interchangeably refers to a poloxamer copolymer-based composition or a hyaluronic acid-based composition as defined herein, optionally further comprising a pharmaceutically acceptable carrier, that is exclusively used in treatment of a medical condition of a mammal, and especially a human, and is aimed at facilitating administration of the active ingredient, i.e., amelogenin, to said mammal. More particularly, the pharmaceutical compositions disclosed herein are useful in treatment of medical conditions wherein administration of amelogenin is, or might be, beneficial, and may thus be used for promoting (i) periodontal or orthopedic soft or hard tissue regeneration; (ii) wound closure, i.e., wound healing; or (iii) skin regeneration and rejuvenation.

The term "tissue regeneration" refers to the process of self-renewal and growth to fill, repair or replace tissue that is damaged or suffers from a disease (Grumezescu and Ficai, 2017). Referring to periodontal soft or hard tissue, the term "tissue regeneration" means supporting regeneration of periodontal attachment, by formation of an acellular extrinsic fiber cementum firmly attached to the underlying root surface with an associated periodontal ligament and aleveolar bone. As assumed by the present inventors, upon administration, the amelogenin comprised within the composition forms a matrix on the root surface; and mesenchymal cells which migrate into the lesion attach to the root surface and start proliferating. Bone formation starts at the treated root surface and subsequently new alveolar bone fill the defect. New attachment with cementum and ligament is formed along the treated root surface. A new functional attachment is achieved with time. Periodontal soft or hard tissue regeneration thus results in an increased gain in clinical attachment, reduction in pocket depth, and bone gain. Referring to orthopedic soft or hard tissue, the term "tissue regeneration" means reconstruction, preferably complete reconstruction, of the subchondral bone and hyaline cartilage in which the chondrocytes are arranged in layers, embedded in their lacunae, and surrounded by extracellular matrix. The level of proteoglycans and collagen type II in the area regenerated, and the thickness of the stained regenerated layers, are similar to that of the normal hyaline cartilage. Referring to orthopedic soft or hard tissue, the term "tissue regeneration" further means reconstruction, preferably complete reconstruction, of injured ligaments and tendons.

The term "wound" as used herein refers to all wound types, including deep wounds and chronic wounds. The term "chronic wound" refers to a wound that exhibits impaired healing parameters interfering with the physiological sequence of events. These wounds tend to prolong and/or halt healing time course, subjecting the wounds to further complications such as recurrent infections and necrosis. The term "wound healing" or "wound closure" used herein interchangeably generally refer to a wound repairing process, i.e., to a complex process in which both the skin and the tissues underlying said skin repair themselves following an injury. In undamaged skin, the epidermis (surface layer) and dermis (deeper layer) form a protective barrier against the external environment. When the barrier is broken or cut, a regulated process including a sequence of biochemical events is set into motion to repair the damage. Said process is often divided into predictable phases including blood clotting (hemostasis), inflammation, tissue growth (proliferation), and tissue remodeling (maturation).

The term "skin regeneration and rejuvenation" as used herein refers to a treatment aimed at inducing collagen production and proliferation, or maintaining skin moisture, thereby correcting textural irregularities like wrinkles, scars such as acne scars, freckles and sunspots, and cellulite, and consequently revealing younger and smoother skin. The efficiency of the amelogenin-containing composition in promoting skin regeneration and rejuvenation results *inter alia* from the ability of amelogenin to help healing compromised extracellular matrix, or act as temporary or surrogate extracellular matrix.

In certain embodiments, the pharmaceutical compositions disclosed are useful for promoting periodontal soft or hard tissue regeneration, e.g., regeneration of a tissue lost due to periodontal disease such as periodontitis or gingival recession, or periodontal trauma such as oral or periodontal wound.

Periodontal disease, also known as gum disease, is a set of inflammatory conditions affecting the tissues surrounding the teeth. In its early stage, called gingivitis, the gums become swollen, red, and may bleed. Later, in its more serious form called periodontitis, the gums can pull away from the tooth, bone may be lost, and the teeth may loosen or fall out. Periodontal disease is generally due to bacteria in the mouth infecting the tissue around the teeth. Risk factors include smoking, diabetes, HIV/AIDS, family history, and certain medications.

Periodontitis is a widespread disease characterized by inflammation-induced progressive damage to the tooth-supporting structures until tooth loss occurs. The regeneration of lost and/or damaged support tissue in the periodontium, including the alveolar bone, periodontal ligament, and cementum, is the purpose of periodontal regenerative therapy and might effectively reduce periodontitis-caused tooth loss (Xu *et al.,* 2019).

Gingival recession, also known as receding gums, is the exposure in the roots of the teeth caused by a loss of gum tissue and/or retraction of the gingival margin from the crown of the teeth. Gum recession is a common problem in adults over the age of 40, but it may also occur earlier and even from the age of a teenager. Gingival recession may exist with or without concomitant decrease in crown-to-root ratio (recession of alveolar bone).

In other embodiments, the pharmaceutical compositions disclosed are useful for promoting orthopedic soft or hard tissue regeneration, e.g., regeneration of bone, cartilage, ligaments, or tendons.

In particular such embodiments, the compositions are useful for treatment of intrabony defects, furcation defects, or recession defects. In other particular such embodiments, the compositions are useful for treatment of articular cartilage defects, e.g., in the knee or ankle joint, cartilage or osteochondral defects, e.g., in the knee or ankle, or mild to moderate osteoarthritis including multiple defects, thereby repairing joint surface lesions, or cartilage focal lesions. In still other particular such embodiments, the compositions are useful for treatment of anterior cruciate ligament injury, Grade II and III acute lateral ankle ligament injury, or scapholunate interosseous ligament injury / scapholunate ligament tear / torn wrist ligaments. In further particular such embodiments, the compositions are useful for treatment of tendon injury, lateral epicondylitis, flexor tendon injury, lesions of the fingers' flexor tendons, injured tendon of the ankle, non-ruptured tendon injuries, or tendinopathy.

In further embodiments, the pharmaceutical compositions disclosed are useful for promoting wound closure, i.e., wound healing, e.g., for assisting in dermal repair and regeneration, or reducing scarring tissue.

In still further embodiments, the pharmaceutical compositions disclosed are useful for promoting skin regeneration and rejuvenation, i.e., inducing collagen production and proliferation, or maintaining skin moisture, for improved skin appearance and wrinkle mitigation.

The medical compositions may be prepared by any suitable techniques, e.g., as described in Remington: The Science and Practice of Pharmacy, 19th Ed., 1995. The compositions can be prepared, e.g., by uniformly and intimately bringing the active agents into association with a liquid carrier, a finely divided solid carrier, or both, and then, if necessary, shaping the product into the desired formulation. Particular procedures for the preparation of such compositions are disclosed in the Experimental section hereinafter. The compositions may be in the form of a liquid (e.g., solution, emulsion, or suspension), or gel, and may further include pharmaceutically acceptable fillers, carriers, diluents or adjuvants, and other inert ingredients and excipients.

Pharmaceutical compositions as disclosed herein may be formulated for any suitable route of administration, but they are preferably formulated for topical administration, intradermal administration, or intraarticular administration (i.e., joint injection including injection to an injured ligament).

The references to methods of treatment in paragraphs [0072], [0073], [0077], [0078] and [0079] of this description are to be interpreted as references to the compounds / pharmaceutical compositions of the present invention for use in a method for treatment of the human (or animal) body by therapy

In a further aspect, disclosed herein is a method for promoting (i) periodontal or orthopedic soft or hard tissue regeneration; (ii) wound closure, i.e., wound healing; or (iii) skin regeneration and rejuvenation, in a subject in need thereof, said method comprising administering to a site in said subject a medical composition as disclosed herein, i.e., a poloxamer copolymer-based composition or a hyaluronic acid-based composition according to any one of the embodiments above.

In certain embodiments, the method disclosed is for promoting periodontal soft or hard tissue regeneration, e.g., regeneration of a tissue lost due to periodontal disease such as periodontitis or gingival recession, or periodontal trauma such as oral or periodontal wound. Such a method may comprise administering said medical composition together with a bone graft implant and/or a membrane for guided tissue and/or bone regeneration (both to the same site in the subject).

Guided bone regeneration and guided tissue regeneration are dental surgical procedures that use barrier membranes to direct the growth of new bone and gingival tissue at sites with insufficient volumes or dimensions of bone or gingiva for proper function, esthetics or prosthetic restoration. Guided bone regeneration typically refers to ridge augmentation or bone regenerative procedures; and guided tissue regeneration typically refers to regeneration of periodontal attachment.

Barrier membranes for use in such procedures should be biocompatible with the host tissue such that they do not induce adverse effect; and able to maintain a space for cells from surrounding bone tissue to migrate for stable time duration, while preventing fibrous tissue that delay bone formation from invading the defect site. In addition, such membranes should have proper physical properties to allow the healing process, including protection of the underlying blood clot; and an adequate degradation time matching the regeneration rate of bone tissue, so as to avoid a secondary surgical procedure to remove the membrane (Zhang *et al.,* 2013).

Non-limiting examples of membranes for guided tissue and/or bone regeneration include non-resorbable membrane made of polytetrafluoroethylene (PTFE), as well as resorbable membranes made of natural collagen or synthetic polylactide, polyglycolide, polylactic-glycolic acid (PLGA), and the like.

In other embodiments, the method disclosed is used for promoting orthopedic soft or hard tissue regeneration, e.g., regeneration of bone, cartilage, ligaments, or tendons. In particular such embodiments, the method is used for treatment of intrabony defects, furcation defects, or recession defects. In other particular such embodiments, the method is used for treatment of articular cartilage defects, e.g., in the knee or ankle joint, cartilage or osteochondral defects, e.g., in the knee or ankle, or mild to moderate osteoarthritis including multiple defects, thereby repairing joint surface lesions, or cartilage focal lesions. In still other particular such embodiments, the method is used for treatment of anterior cruciate ligament injury, Grade II and III acute lateral ankle ligament injury, or scapholunate interosseous ligament injury / scapholunate ligament tear / torn wrist ligaments. In further particular such embodiments, the method is used for treatment of tendon injury, lateral epicondylitis, flexor tendon injury, lesions of the fingers' flexor tendons, injured tendon of the ankle, non-ruptured tendon injuries, or tendinopathy. Such a method may comprise administering said medical composition together with a hyaluronic acid-based product (both to the same site in the subject). Examples of hyaluronic acid-based products include, without limitation, Euflexxa (sodium hyaluronate intra-articular injection, 1%), Hyalgan^{®} (sodium hyaluronate Intra-Articular Injection), Supartz (sodium hyaluronate solution), Monovisc^{™} (high molecular weight hyaluronan injection), Orthovisc^{®} (high molecular weight injectable hyaluronic acid), Gel-One^{®} (cross-linked hyaluronate viscoelastic hydrogel), and Synvisc-One^{®} (hylan G-F 20).

In further embodiments, the method disclosed is used for promoting wound healing, e.g., for assisting in dermal repair and regeneration, or reducing scarring tissue.

In still further embodiments, the method disclosed is used for promoting skin regeneration and rejuvenation, i.e., inducing collagen production and proliferation, or maintaining skin moisture, for improved skin appearance and wrinkle mitigation.

The term "subject" as used herein refers to any mammal, e.g., a human, non-human primate, horse, ferret, dog, cat, cow, and goat. In a preferred embodiment, the term "subject" denotes a human, i.e., an individual.

In yet a further aspect, the present invention relates to a medical composition as defined above, i.e., a poloxamer copolymer-based composition or a hyaluronic acid-based composition, for use in promoting (i) periodontal or orthopedic soft or hard tissue regeneration; (ii) wound closure, i.e., wound healing; or (iii) skin regeneration and rejuvenation.

The poloxamer copolymer-based composition, as defined in any one of the embodiments above, is in the form of a liquid under refrigerated conditions, i.e. at a temperature of about 2-8°C, and upon warming to body temperature, i.e., about 37°C, solidifies into a viscous gel. This composition may thus be packed, e.g., in a vial, or alternatively in a suitable sealed syringe, wherein the amount of liquid composition in said syringe is sufficient for treating either a sole site or a varying number of sites in the subject.

The sealed syringe may be equipped with a blunt needle, suitable for applying, i.e., topically administering, said composition into, e.g., a periodontal pocket / gingival pocket / pocket resulting from peri-implantitis, or for applying said composition on or into an oral or periodontal wound, wherein the amount of the composition in the syringe is sufficient for applying into a sole site, e.g., gingival pocket, or more. Such a syringe may be equipped with 25G needle or tip for optimal injection; however, smaller or larger gauge can be used as well. The syringe is best operated at either ambient or below ambient temperature where the viscosity is low enough to allow precise and controlled delivery without exerting excessive pressure. At this temperature, a physician can deliver the right amount of composition directly to the targeted site, e.g., dental defect, articular defect, or wound surface), where it will turn into gel that will adhere and stay inside the target site. Upon gelation, the highly viscous structure prevents leakage to the surrounding tissue, and controls the release of the therapeutic agent, i.e., amelogenin, to the injured site, in a sustained manner. Alternatively, the liquid composition may be applied into the gingival pocket or onto the oral or periodontal wound using an applicator.

In yet another aspect, the present invention thus relates to a kit comprising a poloxamer copolymer-based composition according to any one of the embodiments above, and a delivery mean, e.g., a syringe or an applicator, for administering or applying said composition to a site in a subject in need of periodontal or orthopedic soft or hard tissue regeneration, wound closure, or skin regeneration and rejuvenation.

The delivery mean included in the kit disclosed herein may be any mean capable of administering or applying a predetermined amount of a liquid thermosensitive polymer-based composition as defined herein to a site in a subject to be treated, which is in need of periodontal or orthopedic soft or hard tissue regeneration, wound closure, or skin regeneration and rejuvenation, e.g., an applicator or a syringe optionally with a blunt needle. In particular embodiments, the kit comprises a syringe with a blunt needle, capable of administering one or more doses of a liquid thermosensitive polymer-based composition as defined herein to a periodontal pocket / gingival pocket / pocket resulting from peri-implantitis, or on or into an oral or periodontal wound.

In contrast to the poloxamer copolymer-based composition, the hyaluronic acid-based composition disclosed herein is in the form of a gel. This composition may thus be provided as ready for use, or alternatively, as two separate compositions to be mixed before use, wherein one of said compositions comprises a high molecular weight hyaluronic acid or a salt thereof, and the other one of said composition comprises amelogenin that is stabilized.

Thus, further disclosed herein is a kit comprising: (i) a delivery mean, e.g., a syringe, or an applicator, for administering a medical composition in a form of a gel into a site in a subject in need of periodontal or orthopedic soft or hard tissue regeneration, wound closure, or skin regeneration and rejuvenation; and (ii) either (a) a pharmaceutical HA-amelogenin composition according to any one of the embodiments above; or (b) a composition A in the form of a gel, comprising a high molecular weight hyaluronic acid or a salt thereof, and a sugar; and a composition B in the form of a liquid, comprising amelogenin, a sugar, an amino acid, and an antioxidant, wherein upon mixing said composition A with said composition B, a medical composition in a form of a gel is obtained, wherein said medical composition has a pH of 3.8 to 5.5, and comprises said hyaluronic acid or a salt thereof in an amount of 0.5% to 10%, said amelogenin in an amount of 0.005% to 3% by weight, a sugar in an amount of 0.05% to 5% by weight, said amino acid in an amount of 0.05% to 5% by weight, and said antioxidant composition in an amount of 0.01% to 2% by weight; and (iii) instructions to mix said composition A, when present, with said composition B so as to obtain said medical composition, and consequently administering said pharmaceutical composition to a site in a subject in need of periodontal or orthopedic soft or hard tissue regeneration, or wound closure.

Unless otherwise indicated, all numbers expressing quantities of ingredients, pH values, and so forth, used in the present description and claims are to be understood as being modified in all instances by the term "about". Accordingly, unless indicated to the contrary, the numerical parameters set forth in this description and attached claims are approximations that may vary by up to plus or minus 10% depending upon the desired properties sought to be obtained by the present invention.

The invention will now be illustrated by the following non-limiting Examples.

### EXAMPLES

Formulations of amelogenin (e.g., X-linked recombinant human amelogenin, rAmelX) should be safe, sterile, stable, and functional for employment as local injection that will induce regeneration in traumatized periodontal and orthopedic tissues.

### Materials and Methods

Various formulations were prepared, first as placebo, i.e., without the active agent, and then with the rAmelX protein. Formulations that passed the requirement for the placebo preparations were prepared with the protein and evaluated in stability studies. The following parameters were evaluated: appearance, assay for rAmelX content, impurities and degradation products, gelation (inversion test), and pH.

***Materials.*** Human recombinant amelogenin (rAmelX) and other reagents referred to herein were purchased from Merck-Sigma.

***Viscosity measurement.*** The viscosity of selected poloxamer copolymer-based compositions was measured with Rheometer MCR 72 (Anton Paar GmbH) using PP25 stainless parallel plate rotor (flat rotor, diameter 25 mm), and measuring position with gap of 0.25 mm, at 6°C under shear rate of 1000/sec, or at 37°C under shear rate of 1/sec.

***rAmelX ("the protein") stock solution preparation.*** Protein stock solution preparations, for preparing various hyaluronic acid (HA)-based compositions and poloxamer copolymer (PX)-based compositions were prepared by directly dissolving the protein at a desired concentration in buffer acetate, pH 3.8-4, comprising the excipients listed in **Table 1** and **Table 2,** respectively.

***Final formulations.*** The protein-containing formulation was prepared in two steps. First, a protein solution preparation as shown in **Table 1** or **Table 2** was prepared and sterilized using 0.2 µm filter, and said preparation was then mixed with a concentrated HA or PX solution, at the desired ratio. The concentrated HA or PX solution was sterilized by an autoclave at 121°C before mixing with the sterile protein solution preparation.

**Table 1. HA-based formulations matrix design**

| **ID** | **Amount in final formulation (mg/ml)** | | | | | | | **Amount in stock protein solution (mg/ml)** | | | | | | |
|---|---|---|---|---|---|---|---|---|---|---|---|---|---|---|
| | Mannitol | Tre | Gln | Met | Lys | Gly | F-68 | Mannitol | Tre | Gln | Met | Lys | Gly | F-68 |
| **HA1** | 9 | | | | | | | 9 | | | | | | |
| **HA2** | 9 | 10 | | | | | | 9 | 20 | | | | | |
| **HA14** | 9 | 10 | 7 | 1 | | | | 9 | 20 | 14 | 2 | | | |
| **HA15** | 9 | 10 | | 1 | 73 | | | 9 | 20 | | 2 | 146 | | |
| **HA16** | 9 | 10 | | 1 | | 4 | | 9 | 20 | | 2 | | 8 | |
| **HA17** | 9 | | | | | | 2 | 9 | | | | | | 4 |
| **HA18** | 9 | 10 | | 1 | | 4 | 2 | 9 | 20 | | 2 | | 8 | 4 |
| **HA19** | 9 | | | | | | 3 | 9 | | | | | | 6 |

***Accelerated stability study.*** Protein stability was examined following incubation of the tested formulations in a stability chamber at 40°C/75% relative humidity, 25°C/60% relative humidity, or refrigeration conditions (2-8°C). Protein analysis was performed using Reverse-Phase High Pressure Liquid Chromatography (RP-HPLC) as follows: Mobile phase A: 0.1% trifluoroacetic acid (TFA); mobile phase B: 0.1% TFA in acetonitrile; HPLC column - C4/S 5µm/20nm, 250×4.6 mm plus Guard column C4 10×4mm, injection volume of 10 µL, flow rate of 1 ml/min, and UV detector at wavelength of 215 nm.

**Table 2. Poloxamer-based formulations matrix design (the effect on gelation was measured by tube inversion test following incubation at 29-31°C for 5 minutes)**

| **ID** | | **PX (%)** | **Mucoadhesive amount (%)** | | | | **20 mM buffer acetate + excipient (mg/ml)** | | | | | | **A** | **B** |
|---|---|---|---|---|---|---|---|---|---|---|---|---|---|---|
| | | | Oligo HA | PGA | XG | GA | NaCl | Mannitol | Trehalose | Gln | Met | Gly | | |
| PX | 1 | 22.5 | 0.6 | | | | | | | | | | No | |
| | 2 | 22.5 | 1.5 | | | | | | | | | | No | |
| | 3 | 26.25 | | | | | | | | | | | **Yes** | **Yes** |
| | 4 | 26.25 | 0.7 | | | | | | | | | | Yes | No^{a} |
| | 5 | 26.25 | 1.75 | | | | | | | | | | No | |
| PX0 | 1 | 22.5 | 0.6 | | | | | | 10 | | | | No | |
| | 2 | 22.5 | 1.5 | | | | | | 10 | | | | No | |
| | 3 | 26.25 | 0.7 | | | | | | 10 | | | | Yes | |
| | 4 | 26.25 | 1.75 | | | | | | 10 | | | | No | |
| PX1 | 1 | 22.5 | 0.075 | | | | | 9 | | | | | No | No^{a} |
| | 2 | 22.5 | 0.3 | | | | | 9 | | | | | No | No^{a} |
| | 3 | 22.5 | 0.6 | | | | | 9 | | | | | No | |
| | 4 | 22.5 | 1.5 | | | | | 9 | | | | | No | |
| | 5 | 26.25 | 0.088 | | | | | 9 | | | | | Yes | Yes^{a} |
| | 6 | 26.25 | 0.35 | | | | | 9 | | | | | Yes | Yes^{a} |
| | 7 | 26.25 | 0.7 | | | | | 9 | | | | | Yes | No^{a} |
| | 8 | 26.25 | 1.75 | | | | | 9 | | | | | No | |
| PX2 | 1 | 26.25 | 0.7 | | | | | 9 | 5 | | | | No | |
| PX3 | 1 | | | | | | | 9 | | 7 | | | c | |
| PX4 | 1 | 26.25 | 0.7 | | | | | 9 | | | 1 | | No | |
| PX5 | 1 | 26.25 | 0.7 | | | | | 9 | | | | 4 | No | |
| PX6 | 1 | | | | | | | 9 | | 7 | 1 | | ^{c} | |
| PX7 | 1 | 26.25 | 0.7 | | | | | 9 | | | 1 | 4 | No | |
| PX8 | 1 | | | | | | | 3 | 3 | 5 | 1 | | ^{c} | |
| PX9 | 1 | 22.5 | 0.075 | | | | | 3 | 3 | | 1 | 4 | No | No^{a} |
| | 2 | 22.5 | 0.3 | | | | | 3 | 3 | | 1 | 4 | No | No^{a} |
| | 3 | 26.25 | 0.08 | | | | | | | | | | Yes | Yes^{a} |
| | 4 | 26.25 | 0.35 | | | | | 3 | 3 | | 1 | 4 | Yes | Yes^{a} |
| | 5 | 26.25 | 0.7 | | | | | 3 | 3 | | 1 | 4 | Yes | Yes^{a} |
| PX10 | 1 | 22.5 | | | | | | | 10 | | | | No | |
| PX12 | 1 | 22.5 | | | | | | | 5 | | | | No | |
| PX13 | 1 | 22.5 | | | | | | 9 | | | | | No | |
| PX14 | 1 | 26.25 | | | | | | | 10 | | | | **Yes** | **Yes** |
| PX15 | 2 | 26.25 | | | | | | | 5 | | 1 | 4 | **Yes** | **Yes** |
| | 14 | 22.5 | | | | 0.375 | | | 5 | | 1 | 4 | No | |
| | 15 | 22.5 | | | | 0.188 | | | 5 | | 1 | 4 | No | |
| | 16 | 26.25 | | | | 0.438 | | | 5 | | 1 | 4 | Yes | Yes^{a} |
| | 17 | 26.25 | | | | 0.219 | | | 5 | | 1 | 4 | Yes | Yes^{a} |
| PX-16 | 1 | 26.25 | | | | | | | 5 | | 2 | 4 | **Yes** | **Yes** |
| PX-17 | 1 | 26.25 | | | | | | | 5 | | 2 | 3 | **Yes** | **Yes** |
| PX-18 | 1 | 26.25 | | | | | | | 7 | | 3 | | **Yes** | **Yes** |

| | | | | | | | | | | | | | | |
|---|---|---|---|---|---|---|---|---|---|---|---|---|---|---|
| **XG** - xanthan gum; **GA** - gum acacia; **^{a}** turbid; **^{b}** precipitation; **^{c}** excipient buffer not dissolved; **^{d}** stock gel (30% PX with 0.2% or 0.3% XG) is not liquid below 30°C; **A** - gelation in the tested conditions (placebo); **B** - gelation in the tested conditions (amelogenin-containing formulation). | | | | | | | | | | | | | | |

***Gelation determination.*** The effect on gelation of the poloxamer-based formulations was determined by tube inversion test following incubation of the tested formulation at 29-31°C for 5 minutes.

### Example 1. Stability of HA-rAmelX gel formulations

A key point in developing of HA-based rAmelX gel formulations is succeeding in stabilizing the rAmelX at liquid/gel-based formulation.

1 mg/ml rAmelX was dissolved in 40 mM buffer acetate, pH 4, comprising the excipients listed in **Table 1,** and sterilized by filtration. The protein solution obtained was then mixed at 1:1 ratio with a sterilized concentrated HA gel solution containing 4% HA, 0.9% mannitol, and water.

The results of the preliminary accelerated stability study are presented in **Table 3,** showing rAmelX assay (% of nominal) at 40°C/75% relative humidity and under refrigeration conditions. As shown, rAmelX was not stabilized in formulations HA-1, HA-2, HA-14, and HA-18. Significant lost (>40%) was observed after 1-month (1M) storage at 40°C. On the other hand, formulation HA-16 which contained specific amounts of mannitol, trehalose, glycine and methionine exhibited promising results, wherein rAmelX was stable (rAmelX content was reduced by not more than 15%) for one month in 40°C predicting at least 12 months shelf life under refrigeration.

**Table 3. Accelerated stability study of selected HA-based formulations**

| | | | **rAmelX assay (% of nominal)** | | | | | | | |
|---|---|---|---|---|---|---|---|---|---|---|
| **ID** | **pH** | **Initial** | **Refrigeration, 2-8°C** | | | | | **Accelerated, 40°C/75% relative humidity** | | |
| | | | Day 10-16 | 1M | Day 41-44 | Day 52 | Day 69 | Day 10-16 | 1M | Day 44 |
| HA-1 | 4.0 | 97.8 | 96.4 | 79.0 | | | | 56.5 | 38.4 | |
| HA-2 | 4.1 | 90.7 | 88.3 | 81.1 | | | | 65.4 | 55.8 | |
| HA-14 | 3.8 | 113.4 | 76.2 | 82.1 | | | | 55.8 | 46.5 | |
| HA-15 | ND, precipitated | | | | | | | | | |
| HA-16 (n=2) | 4.0 | 99.9 | 100.4 | 105.5 | 106.8 | 95.3 | 94.8 | 94.0 | 84.9 | |
| HA-17 (n=2) | 4.0 | 103.5 | 100.8 | 104.9 | 94.83 | | | 83.8 | 81.9 | 80 |
| HA-18 (n=2) | 4.0 | 107.5 | 110.2 | 106.3 | | | | 67.3 | 45.4 | |
| HA-19 | 3.9 | 105.0 | 111.3 | | | | | 90.7 | | |

### Example 2. Stability of poloxamer-based gel (PX-based gel)

A key point in developing of thermosensitive gel-based rAmelX formulations is succeeding in stabilizing the rAmelX while maintaining the thermo-sensitivity of the formulation at the clinical conditions. The first condition for achieving the goal is by demonstrating that the sterilization of the poloxamer stock solution does not hamper its thermo-sensitivity. **Table 4** shows that the thermo-sensitivity of various concentrated poloxamer-based solutions following sterilization by an autoclave was maintained.

4 mg/ml rAmelX was dissolved in 160 mM buffer acetate, pH 3.8-4, comprising the excipients listed in **Table 2** concentrated ×8, and sterilized by filtration. The protein solution obtained was then mixed at 1:7 ratio with a sterilized concentrated poloxamer solution containing 30% poloxamer (PX) 407 and water to obtain a final concentration of 0.5 mg/ml rAmelX and 26.25% poloxamer in 20 mM acetate buffer pH 3.8-5.0.

rAmelX stability was examined following incubation of the thermosensitive formulations in a stability chamber at 40°C/75% relative humidity, 25°C/60% relative humidity, or refrigeration conditions (2-8°C). Analysis was performed as described above. The results of the stability study of selected formulations are presented in **Table 5,** showing rAmelX assay (% of nominal).

Formulation PX26-15-2 was demonstrated to be the most promising, showing stability for at least 12 months under refrigerated conditions; five months at room temperature; and at least one month at 40°C. The stability of formulation PX26-15-2 was further evaluated, using different rAmelX concentrations, and the data are summarized in **Table 6.** As shown, the formulation was stable for at least one month under accelerated conditions at 40°C, at least three months under accelerated conditions at 25°C, and at least 12 months under long term conditions at 2-8°C.

The viscosity of formulation PX26-15-2, lot BCM-30-01231 at release, containing 0.5 mg/ml rAmelX, was measured as described above, and found to be 118 cP vs. 154,350 cP, at 6°C and 37°C, respectively.

***Conclusions.*** As found, sugar, e.g., trehalose, only was not sufficient for stabilizing the formulation (formulation PX26-14 was found to be unstable), and addition of the antioxidant methionine was essential. Formulations PX26-15-2 and PX26-16, both containing methionine (as antioxidant) and glycine, were stable for at least 12 months at 2-8°C (long term storage). When tested under accelerated conditions (at 25°C and 40°C), formulation PX26-15-2 was found to be slightly better and was therefore subject for further stability experiments and selected for testing in animal studies. Glycine was found to contribute to rAmelX stability and to further act as a taste masking for dental preparations.

**Table 4. Effect of sterilization on thermo-sensitivity of concentrated gel solutions**

| **Sample** | **Gelation before sterilization** | **Gelation after sterilization** |
|---|---|---|
| 30% PX 407 | Yes | Yes |
| 30% PX 407, 0.1% oligo HA, 0.9% mannitol | Yes | Yes |
| 30% PX 407, 0.1% oligo HA, 0.3% mannitol | Yes | Yes |
| 30% PX 407, 0.4% oligo HA, 0.9% mannitol | Yes | Yes |
| 30% PX 407, 0.4% oligo HA, 0.3% mannitol | Yes | Yes |
| 30% PX 407, 0.8% oligo HA, 0.9% mannitol | Yes | Yes |
| 30% PX 407, 0.8% oligo HA, 0.3% mannitol | Yes | Yes |
| 30% PX 407, 2% oligo HA | Yes | Yes |
| 30% PX 407, 0.25% PGA | Yes | Yes |
| 30% PX 407, 0.1% PGA | Yes | Yes |
| 24% PX 407, 0.3% xanthan gum | Yes | Yes |
| 24% PX 407, 0.2% xanthan gum | Yes | Yes |
| 30% PX 407, 0.5% gum acacia | Yes | Yes |
| 30% PX 407, 0.25% gum acacia | Yes | Yes |

**Table 5. Accelerated stability study of selected PX-based formulations**

| **ID** | **Parameter** | **Assay (% of nominal)** | | | | | | | | | | |
|---|---|---|---|---|---|---|---|---|---|---|---|---|
| | | **Initial** | **25°C/60% RH, M** | | | | | | **40°C/75% RH, M** | | | |
| | | | 1 | 2 | 3 | 4 | 5 | 6 | 0.5 | 1 | 1.5 | 2 |
| PX26-14 | pH | N/P | N/P | | 4.4 | 4.4 | 4.4 | N/P | N/P | | N/P | 4.4 |
| | Gelation | Conform | N/P | | | | | | N/P | | | N/P |
| | Assay | 107 | 99 | 94 | <LOD | | | | <LOD | | | <LOD |
| | IDD | <LOD | <LOD | | 100.0 | | | | 100.0 | | | 0.0 |
| PX26-15-2 | pH | 4.7 | N/P | | 4.7 | N/P | | 4.85 | N/P | | | 4.7 |
| | Gelation | Conform | | | N/P | | | Conform | | | | N/P |
| | Assay | 108 | 106 | 102 | 107 | 101 | 103 | 73 (turbid) | 110 | 101 | 96 | 90 |
| | IDD | <LOD | <LOD | | 6.2 | 4.8 | 8.4 | 27.1 | <LOD | | 1.9 | 13.2 |
| PX26-16 | pH | 4.6 | N/P | | 4.7 | N/P | | 4.9 | N/P | | N/P | |
| | Gelation | Conform | N/P | | | | | Conform | N/P | | | |
| | Assay | 102 | 98 | 90 | 91 | 84 | 88 | 83 (turbid) | 104 | 89 | | |
| | IDD | <LOD | <LOD | | 6.6 | 4.2 | 8.1 | 22.6 | <LOD | 3.9 | | |
| PX26-18 | pH | 4.5 | N/P | | 4.7 | N/P | N/P | 4.8 | N/P | | | N/P |
| | Gelation | Conform | N/P | | | | | Conform | N/P | | | |
| | Assay | 120 | 123 | 112 | 115 | 105 | 105 | 99 (turbid) | 125 | 110 | 105 | |
| | IDD | <LOD | <LOD | | 5.2 | 4.2 | 17.5 | 32.4 | <LOD | 4.5 | 7.2 | |

**Table 5. (continued)**

| **ID** | **Parameter** | **Assay (% of nominal)** | | | | | | |
|---|---|---|---|---|---|---|---|---|
| | | **Initial** | **2-8°C, M** | | | | | |
| | | | 1 | 2 | 3 | 6 | 9 | 12 |
| PX26-14 | pH | N/P | N/P | | 4.4 | 4.8 | N/P | |
| | Gelation | Conform | N/P | | | Conform | | |
| | Assay | 107 | 99 | 96 | 97 | <LOD | | |
| | IDD | <LOD | <LOD | | | 100.0 | | |
| PX26-15-2 | pH | 4.7 | N/P | | 4.7 | 4.9 | 4.9 | 4.3 |
| | Gelation | Conform | | | N/P | Conform | | |
| | Assay | 108 | 105 | 97 | 103 | 100 | 97 | 99 |
| | IDD | <LOD | <LOD | | | | | |
| PX26-16 | pH | 4.6 | N/P | | 4.7 | 4.8 | 4.7 | 4.3 |
| | Gelation | Conform | N/P | | | Conform | N/P | |
| | Assay | 102 | 99 | 94 | 92 | 93 | 98 | 99 |
| | IDD | <LOD | <LOD | | | | | |
| PX26-18 | pH | 4.5 | N/P | | 4.7 | 4.7 | 4.9 | 4.3 |
| | Gelation | Conform | N/P | | | Conform | N/P | Conform |
| | Assay | 120 | 123 | 117 | 123 | 121 | 120 | 61 (turbid) |
| | IDD | <LOD | <LOD | | | | | |

| | | | | | | | | |
|---|---|---|---|---|---|---|---|---|
| *LOD - level of detection *N/P - Not performed * IDD - Impurities and degradation products * conform - to gelation test at 37°C | | | | | | | | |

### Example 3. Evaluation of healing in periodontal type defects using rAmelX gel formulation in minipigs

The safety and effectiveness of the rAmelX formulation PX26-15-2 in the management of recession and intrabony periodontal defects was evaluated in a minipig model. The regenerative capability of the product on both bone and soft tissue was assessed.

***Study protocol.*** Three inbred Sinclair miniature female pigs, 18 months old and weighing 50-60 kg, were used. The study included three stages referred to herein as Surgery I, IIa and IIb.

*Surgery I.* Tooth extraction in the mandible. The third premolars (P3) of both mandibular quadrants were extracted and the extraction sites were left to heal for at least four weeks. The remaining dentition received oral prophylaxis with chlorhexidine solution after the extraction procedure.

**Table 6. Stability studies of formulation PX26-15-2 with various rAmelX concentrations**

| **rAmelX (mg/ml)** | **Test** | **Assay (% of nominal)** | | | | | | | | | |
|---|---|---|---|---|---|---|---|---|---|---|---|
| | | **Initial** | **2-8°C, M** | | | | **25°C/60% RH, M** | | | **40°C/75% RH, M** | |
| | | | **3** | **6** | **9** | **12** | **1** | **2** | **3** | **0.5** | **1** |
| 0.25¹ | pH | N/P | 4.4 | 4.4 | 4.4 | | N/P | 4.4 | 4.7 | N/P | |
| | Gelation | conform | N/P | conform | N/P | | | N/P | | | |
| | Assay | 108 | 107 | 103 | 98 | | 106 | 109 | 112 | | |
| | IDD | <LOD | 1.8 | 1.3 | 1.9 | | <LOD | | 5.3 | | |
| 0.5¹ | pH | N/P | 4.4 | 4.4 | N/P | | N/P | 4.4 | 4.4 | N/P | |
| | Gelation | conform | N/P | conform | | | | N/P | | | |
| | Assay | 113 | 110 | 100 | | | 105 | 111 | N/P | | |
| | IDD | <LOD | 2.0 | 1.5 | | | <LOD | | | | |
| 1¹ | pH | N/P | 4.4 | 4.4 | 4.4 | | N/P | 4.4 | 4.4 | N/P | |
| | Gelation | conform | N/P | Conform | N/P | | | N/P | | | |
| | Assay | 107 | 104 | 103 | 100 | | 103 | 104 | N/P | | |
| | IDD | <LOD | <LOD | <LOD | <LOD | | <LOD | | | | |
| 0.5² | pH | N/P | N/P | 4.7 | 4.4 | 4.4 | N/P | 4.4 | N/P | N/P | 4.4 |
| | Gelation | conform | | conform | N/P | conform | | N/P | | | N/P |
| | Assay | 106 | 108 | 107 | 106 | 93 | 103 | 104 | 105 | 101 | 85 |
| | IDD | <LOD | <LOD | <LOD | <LOD | <LOD | <LOD | 2.3 | 2.6 | <LOD | 4.0 |
| 0.5³ | pH | 4.1 | 4.8 | N/P | | | 4.1 | N/P | 4.9 | N/P | 4.1 |
| | Gelation | conform | conform | N/P | | | N/P | | conform | | conform |
| | Assay | 110 | 113 | 110 | | | 112 | 111 | 112 | | 102 |
| | IDD | <LOD | <LOD | <LOD | | | <LOD | | | | 4.3 |
| 0.5⁴ | pH | 4.6 | 4.8 | 4.4 | | | N/P | | 4.7 | 4.4 | 4.7 |
| | Gelation | conform | conform | conform | | | | | conform | N/P | conform |
| | Assay | 101 | 104 | 103 | | | 102 | | 102 (turbid) | 99 | 96 |
| | IDD | <LOD | <LOD | 5.2 | | | <LOD | | <LOD | <LOD | 3.5 |
| 0.5⁵ | pH | 4.7 | 4.3 | | | | N/P | | 4.4 | N/P | 4.7 |
| | Gelation | conform | conform | | | | N/P | | | N/P | conform |
| | Assay | 115 | 108 | | | | 112 | 114 | 110 | 112 | 115 |
| | IDD | <LOD | <LOD | | | | <LOD | | | <LOD | |
| 0.5⁶ | pH | 4.7 | 4.8 | | | | N/P | N/P | 4.7 | N/P | 4.4 |
| | Gelation | conform | conform | | | | N/P | N/P | conform | N/P | conform |
| | Assay | 114 | 111 | | | | 114 | 113 | 113 | 109 | 111 |
| | IDD | <LOD | <LOD | | | | <LOD | | 2.6 | <LOD | |

| | | | | | | | | | | | |
|---|---|---|---|---|---|---|---|---|---|---|---|
| ¹ Batch ID: EXPC-123012020 ² Batch ID: EXLum-130012020 ³ Batch ID: EXPC-G11052020 ⁴ Batch ID: EXPC-P01062020 ⁵ Batch ID: EXCon48-103082020 ⁶ Batch ID: EXCon48-204082020 * LOD - level of detection * N/P - Not performed * IDD - Impurities and degradation products * conform - to gelation test at 37°C | | | | | | | | | | | |

*Surgery IIa.* Acute defect creation in the mandible and treatment. Following intrasulcular incisions, mucoperiosteal flaps was raised and acute "box-type" 2-wall intrabony defects of approximately 4×4×4 mm was surgically created by leaving the lingual bone wall intact in premolars teeth PM2 and PM4. The defects were created at the mesial aspect of the second premolar and fourth premolar of both mandibular quadrants. Subsequently, the roots were thoroughly scaled to completely remove the root cementum and periodontal ligament. The animals were randomly assigned to an allocation group and treated as specified in the allocation plan: (a) Control sites, carrier only/placebo (12 defects in three miniature pigs); and (b) Test sites, rAmelX gel formulation, 0.5 mg/ml rAmelX (12 defects in three miniature pigs). The tested material was adjusted to the defect. After treatment, the soft tissues were positioned at the pre-surgical level and the wound was closed tension-free.

*Surgery IIb.* Same day as surgery I. Recession coverage in the maxilla and treatment. An intrasulcular incision was performed including the maxillary P2 and P4, with a small releasing vertical incision. A muco-periosteal full-thickness flap was then elevated to expose the buccal alveolar bone. At both teeth (P2 and P4), dehiscence defects measuring 5 mm in depth and 4 mm in width were created apical to the cemento-enamel junction. The root surfaces were then thoroughly scaled and planed. Randomization was performed and the recessions were assigned to either receive rAmelX gel formulation (0.5 mg/ml, test sites) or carrier only (placebo, control sites). The flaps were then sutured and stabilized.

***Retrieving specimens and histology assessment.*** Three months after surgeries IIa-b, the animals were anesthetized. The mandibles and maxillas were removed. Individual bone blocks containing the implanted biomaterials and the surrounding soft and hard tissues were obtained and subsequently fixed. Histological sections were prepared, and the most central sections were chosen for histomorphometric and statistical analysis.

***Study outcomes measures.*** Intrabony defects: Height of new cementum, new bone height, defect height, junctional epithelium height and connective tissue adhesion. Recession defects: Histological description of healed site. Percentage of bone regeneration and soft tissue attachment.

***Results.*** It is expected that there would be significant changes between the control and the experimental in bone gain and clinical attachment level, mobility parameters, and pocket depths, and that following all periodontal tissues, i.e., the cementum, the periodontal ligaments and the alveolar bone, will be regenerated.

### Example 4. Evaluation of rAmelX gel formulation in orthopedic applications

Animal studies evaluating the formulation for orthopedic applications were led and performed by Dr. Amir Haze, Dr. Anat Blumenfeld and Dr. Hani Nevo, the Hebrew University - Hadassah, Jerusalem, Israel.

***Recruitment of mesenchymal stem cells (MSCs) into the injured site between the stumps of transected rat medial collateral ligament (MCL) by rAmelX in the new PX based formulation.*** rAmelX PX based formulation at a concentration of 0.25-0.5 mg/ml recruited MSCs to the injured significantly more than placebo formulation (data not shown).

***Regeneration of osteochondral defect (OCD) by rAmelX PX based formulation in a rat model.*** An OCD was created in the rat knee joint followed by treatment with 0.5 mg/ml rAmelX PX based formulation or placebo formulation. Twelve weeks after the operation, the rats were euthanized, and the regeneration of the defect was evaluated by gross assessment of the size of the defect. It was found that regeneration of rat knee OCD was higher in the treatment group (data not shown).

***Regeneration of OCD using rAmelX in poloxamer based formulation in a goat model.*** A large OCD was created in the weight-bearing area in the left and right medial femoral condyle (MFC) and treated with placebo or rAmelX PX based formulation. Both magnetic resonance imaging (MRI) scans of the first goat, three- and six-months post-surgery, and clinical examination of the post sacrificed goat, revealed that the subchondral bone in the experimental knee healed significantly better than in the control knee, filling of the defect with tissue characterized as partially differentiated hyaline cartilage and fibrocartilage at the level of the joint surface (data not shown). Results of 5 more goats are pending.

### REFERENCES

Bonde J. S., Bulow L., One-step purification of recombinant human amelogenin and use of amelogenin as a fusion partner., PLoS One., 2012, 7, e33269
Bittner S., When quinones meet amino acids: chemical, physical and biological consequences, Amino Acids, 2006, 30, 205-224
Cheng L., Lin Z.K., Shu R., Liu D.L., Zhang X.L., Liu B., Wang J., Tian L., Analogous effects of recombinant human full-length amelogenin expressed by Pichia pastoris yeast and enamel matrix derivative in vitro. Cell Prolif., 2012, 45(5), 456-65
Cochran D.L., Jones A., Heijl L., Mellonig J.T., Schoolfield J., King G.N., Periodontal regeneration with a combination of enamel matrix proteins and autogenous bone grafting. Journal of periodontology, 2003, 74, 1269-1281
Forney-Stevens K.M., Bogner R.H., Pikal M.J., Addition of amino acids to further stabilize lyophilized sucrose-based protein formulations: I. Screening of 15 amino acids in two model proteins. Journal of pharmaceuticalsciences, 2016, 105, 697-704
Grumezescu A.M., Ficai A., Nanostructures for Cancer Therapy, Elsevier, 2017
Jiang D., Liang J., Fan J., Yu S., Chen S., Luo Y., Prestwich G.D., Mascarenhas M.M., Garg H.G., Quinn D.A., Homer R.J., Goldstein D.R., Bucala R., Lee P.J., Medzhitov R., Noble P.W., Regulation of lung injury and repair by Toll-like receptors and hyaluronan NatMed., 2005, 11, 1173-1179
Lekovic V., Camargo P.M., Weinlaender M., Nedic M., Aleksic Z., Kenney E.B., A comparison between enamel matrix proteins used alone or in combination with bovine porous bone mineral in the treatment of intrabony periodontal defects in humans. J. Periodontol., 2000, 71, 1110-6
McBride W.H., Bard J.B., Hyaluronidase-sensitive halos around adherent cells. Their role in blocking lymphocyte-mediated cytolysis. J Exp Med. 1979, 149, 507-515
Mellonig J.T., Enamel matrix derivative for periodontal reconstructive surgery: technique and clinical and histologic case report, Int J Periodontics Restorative Dent., 1999, 19, 8-19
Miron R.J., Bosshardt D.D., Buser D., Zhang Y., Tugulu S., Gemperli A., Dard M., Caluseru O.M., Chandad F., Sculean A., Comparison of the capacity of enamel matrix derivative gel and enamel matrix derivative in liquid formulation to adsorb to bone grafting materials., J Periodontol. 2015, 86, 578-87
Morrison H., Sherman L.S., Legg J., Banine F., Isacke C., Haipek C.A., Gutmann D.H., Ponta H., Herrlich P., The NF2 tumor suppressor gene product, merlin, mediates contact inhibition of growth through interactions with CD44, Genes Dev., 2001, 15, 968-980
Ohtake S., Kita Y., Arakawa T., Interactions of formulation excipients with proteins in solution and in the dried state. Advanced drug delivery reviews, 2011, 63, 1053-1073
Sculean A., Schwarz F., Becker J., Brecx M., The application of an enamel matrix protein derivative (Emdogain®) in regenerative periodontal therapy: a review. Medical Principles and Practice, 2007, 16, 167-180
Shirakata Y., Yoshimoto T., Goto H., Yonamine Y., Kadomatsu H., Miyamoto M., Nakamura T., Hayashi C., Izumi Y., Favorable periodontal healing of 1-wall infrabony defects after application of calcium phosphate cement wall alone or in combination with enamel matrix derivative: a pilot study with canine mandibles. J Periodontol., 2007, 78, 889-898
Sukumar S., Drizhal I., Hyaluronic acid and periodontitis, Acta Medica (Hradec Kralove), 2007, 50, 225-228
Taylor A.L., Haze-Filderman A., Blumenfeld A., Shay B., Dafni L., Rosenfeld E., Leiser Y., Fermon E., Gruenbaum-Cohen Y., Deutsch D., High yield of biologically active recombinant human amelogenin using the baculovirus expression system., Protein Expr Purif., 2006, 45, 43-53
Xu X.Y., Li X., Wang J., He X.T., Sun H.H., Chen F.M., Concise review: periodontal tissue regeneration using stem cells: strategies and translational considerations. Stem cells translational medicine, 2019, 8, 392-403
Zhang Y., Zhang X., Shi B., Miron R.J., Membranes for guided tissue and bone regeneration. Annals of Oral & Maxillofacial Surgery, 2013, 1, 10

## Claims

1. A liquid medical composition comprising a non-biodegradable thermosensitive pharmaceutically acceptable poloxamer copolymer in an amount of 18% to 30% by weight; amelogenin in an amount of 0.005% to 3% by weight; a disaccharide in an amount of 0.05% to 5% by weight; an amino acid selected from alanine, glycine, isoleucine, leucine, proline, valine, and a mixture thereof in an amount of 0.05% to 5% by weight; and an antioxidant which is an o-quinone scavenger selected from ascorbic acid or a salt thereof, ascorbic acid derivative such as ascorbic acid-6-palmitate, methionine, N-acethylmethionine, cysteine, N-acetyl cysteine (NAC), and gluthatione (GSH), in an amount of 0.01% to 2% by weight,
wherein said composition has a pH of 3.8 to 5.5, and a viscosity of 50-2000 millipascal seconds (mPa·sec) at a temperature of about 2-8°C, when measured with Rheometer MCR 72 under shear rate of 1000 sec⁻¹; and upon warming to about 37°C, said composition solidifies into a gel having a viscosity of at least 10000 mPa·sec when measured with Rheometer MCR 72 under shear rate of 1 sec⁻¹.

2. The composition of claim 1, wherein said poloxamer copolymer is poloxamer 407, poloxamer 188, poloxamer 124, poloxamer 237, poloxamer 338, or a mixture thereof.

3. The composition of claim 2, wherein said poloxamer copolymer is poloxamer 407.

4. The composition of claim 3, wherein said poloxamer copolymer is present in said composition in an amount of 21% to 29%, 23% to 28%, or 25% to 27%, by weight.

5. The composition of claim 1, wherein said amelogenin is a natural amelogenin or a recombinant amelogenin.

6. The composition of claim 5, wherein said amelogenin is present in said composition in an amount of 0.01% to 1% by weight.

7. The composition of claim 1, wherein:
(i) said disaccharide is sucrose, trehalose, lactose, maltose, cellobiose, gentiobiose, or a mixture thereof;
(ii) said amino acid is glycine; or
(iii) said antioxidant is L-methionine, N-acethylmethionine, L-cysteine, N-acetyl cysteine (NAC), glutathione (GSH), ascorbic acid, an ascorbate such as sodium ascorbate, an ascorbic acid derivative such as ascorbic acid-6-palmitate, or a mixture thereof.

8. The composition of claim 7, wherein:
(i) said disaccharide is present in said composition in an amount of 0.1% to 2.5% by weight;
(ii) said amino acid is present in said composition in an amount of 0.1% to 1% by weight; or
(iii) said antioxidant is present in said composition in an amount of 0.05% to 1% by weight.

9. The composition of claim 1, wherein said poloxamer copolymer is poloxamer 407; said amelogenin is a recombinant amelogenin; said disaccharide is trehalose; said amino acid is glycine; and said antioxidant is methionine.

10. The composition of claim 9, wherein said poloxamer copolymer is present in said composition in an amount of 21% to 29% by weight; said amelogenin is present in said composition in an amount of 0.01% to 1% by weight; said disaccharide is present in said composition in an amount of 0.1% to 2.5% by weight; said amino acid is present in said composition in an amount of 0.1% to 1% by weight; said antioxidant is present in said composition in an amount of 0.05% to 1% by weight; and said composition has a pH of 4.0 to 5.0.

11. The composition of claim 10, wherein said poloxamer copolymer is present in said composition in an amount of 25% to 27% by weight; said amelogenin is present in said composition in an amount of 0.02% to 0.5% by weight; said disaccharide is present in said composition in an amount of 0.2% to 1% by weight; said amino acid is present in said composition in an amount of 0.2% to 0.5% by weight; and said antioxidant is present in said composition in an amount of 0.1% to 0.5% by weight.

12. The composition of any one of claims 1 to 11, further comprising a mucoadhesive agent such as a low molecular weight hyaluronic acid (HA), propylene glycol alginate (PGA), xanthan gum, gum acacia, a carbomer (polyacrylic acid), chitosan, human serum albumin, gellun gum, guar gum, carrageenan, and a cellulose derivative such as carboxymethyl cellulose and hydroxypropyl methylcellulose; and/or a buffering agent such as acetic acid-, citric acid-, tartaric acid-, lactic acid-, hydrochloric acid-, or hydrogenphosphoric acid-based buffer, diethylamine-based buffer, Britton-Robinson buffer, and Carmody buffer.

13. The composition of any one of claims 1 to 12, for use in promoting (i) periodontal or orthopedic soft or hard tissue regeneration; (ii) wound closure; or (iii) skin regeneration and rejuvenation.

14. The composition for use according to claim 13, wherein said orthopedic soft tissue is cartilage, ligaments, or tendons; said orthopedic hard tissue is bone; or said periodontal soft or hard tissue is a tissue lost due to periodontal disease such as periodontitis or gingival recession, or periodontal trauma such as oral or periodontal wound.

15. The composition for use according to claim 14, for:
(i) treatment of intrabony defects, furcation defects, or recession defects;
(ii) treatment of articular cartilage defects, cartilage or osteochondral defects, or mild to moderate osteoarthritis including multiple defects, thereby repairing joint surface lesions, or cartilage focal lesions;
(iii) treatment of anterior cruciate ligament injury, Grade II and III acute lateral ankle ligament injury, or scapholunate interosseous ligament injury / scapholunate ligament tear / torn wrist ligaments; or
(iv) treatment of tendon injury, lateral epicondylitis, flexor tendon injury, lesions of the fingers' flexor tendons, injured tendon of the ankle, non-ruptured tendon injuries, or tendinopathy

16. The composition for use according to claim 13, wherein said promoting wound closure is assisting in dermal repair and regeneration, or reducing scarring tissue.

17. A kit comprising a liquid medical composition according to any one of claims 1 to 12; and a delivery mean, such as a syringe or an applicator, for administering said composition to a site in a subject in need of periodontal or orthopedic soft or hard tissue regeneration, wound closure, or skin regeneration and rejuvenation.

## Patentansprüche

1. Flüssige medizinische Zusammensetzung umfassend ein nicht biologisch abbaubares wärmeempfindliches pharmazeutisch annehmbares Poloxamer-Copolymer in einer Menge von 18 bis 30 Gew.-%; Amelogenin in einer Menge von 0,005 bis 3 Gew.-%; ein Disaccharid in einer Menge von 0,05 bis 5 Gew.-%; eine Aminosäure ausgewählt aus Alanin, Glycin, Isoleucin, Leucin, Prolin, Valin und einem Gemisch davon in einer Menge von 0,05 bis 5 Gew.-%; und ein Antioxidans, das ein o-Chinon-Fänger ist, ausgewählt aus Ascorbinsäure oder einem Salz davon, Ascorbinsäurederivat, wie z.B. Ascorbinsäure-6-palmitat, Methionin, N-Acethylmethionin, Cystein, N-Acetylcystein (NAC) und Glutathion (GSH), in einer Menge von 0,01 bis 2 Gew.-%,
Wobei die Zusammensetzung einen pH-Wert von 3,8 bis 5,5 und eine Viskosität von 50-2000 Millipascalsekunden (mPa·s) bei einer Temperatur con etwa 2-8 °C, wenn gemessen mit einem Rheometer MCR 72 bei einer Scherrate von 1000 s⁻¹, aufweist; und die Zusammensetzung bei Erwärmen auf etwa 37 °C eine Viskosität von wenigstens 10000 mPa·s, wenn gemessen mit einem Rheometer MCR 72 bei einer Scherrate von 1 s⁻¹, aufweist.

2. Zusammensetzung nach Anspruch 1, wobei das Poloxamer-Copolymer Poloxamer 407, Poloxamer 188, Poloxamer 124, Poloxamer 237, Poloxamer 338 oder ein Gemisch davon ist.

3. Zusammensetzung nach Anspruch 2, wobei das Poloxamer-Copolymer Poloxamer 407 ist.

4. Zusammensetzung nach Anspruch 3, wobei das Poloxamer-Copolymer in der Zusammensetzung in einer Menge von 21 bis 29 Gew.-%, 23 bis 28 Gew.-% oder 25 bis 27 Gew.-% vorhanden ist.

5. Zusammensetzung nach Anspruch 1, wobei das Amelogenin ein natürliches Amelogenin oder ein rekombinantes Amelogenin ist.

6. Zusammensetzung nach Anspruch 5, wobei das Amelogenin in der Zusammensetzung in einer Menge von 0,01 bis 1 Gew.-% vorhanden ist.

7. Zusammensetzung nach Anspruch 1, wobei:
(i) das Disaccharid Saccharose, Trehalose, Lactose, Maltose, Cellobiose, Gentiobiose oder ein Gemisch davon ist;
(ii) die Aminosäure Glycin ist; oder
(iii) das Antioxidans L-Methionin, N-Acethylmethionin, L-Cystein, N-Acetylcystein (NAC), Glutathion (GSH), Ascorbinsäure, ein Ascorbat, wie z.B. Natriumascorbat, ein Ascorbinsäurederivat, wie z.B. Ascorbinsäure-6-palmitat, oder ein Gemisch davon ist.

8. Zusammensetzung nach Anspruch 7, wobei:
(i) das Disaccharid in der Zusammensetzung in einer Menge von 0,1 bis 2,5 Gew.-% vorhanden ist;
(ii) die Aminosäure in der Zusammensetzung in einer Menge von 0,1 bis 1 Gew.-% vorhanden ist; oder
(iii) das Antioxidans in der Zusammensetzung in einer Menge von 0,05 bis 1 Gew.-% vorhanden ist.

9. Zusammensetzung nach Anspruch 1, wobei das Poloxamer-Copolymer Poloxamer 407 ist; das Amelogenin ein rekombinantes Amelogenin ist; das Disaccharid Trehalose ist; die Aminosäure Glycin ist; und das Antioxidans Methionin ist.

10. Zusammensetzung nach Anspruch 9, wobei das Poloxamer-Copolymer in der Zusammensetzung in einer Menge von 21 bis 29 Gew.-% vorhanden ist; das Amelogenin in der Zusammensetzung in einer Menge von 0,01 bis 1 Gew.-% vorhanden ist; das Disaccharid in der Zusammensetzung in einer Menge von 0,1 bis 2,5 Gew.-% vorhanden ist; die Aminosäure in der Zusammensetzung in einer Menge von 0,1 bis 1 Gew.-% vorhanden ist; das Antioxidans ist in der Zusammensetzung in einer Menge von 0,05 bis 1 Gew.-% vorhanden; und die Zusammensetzung einen pH-Wert von 4,0 bis 5,0 aufweist.

11. Zusammensetzung nach Anspruch 10, wobei das Poloxamer-Copolymer in der Zusammensetzung in einer Menge von 25 bis 27 Gew.-% vorhanden ist; das Amelogenin in der Zusammensetzung in einer Menge von 0,02 bis 0,5 Gew.-% vorhanden ist; das Disaccharid in der Zusammensetzung in einer Menge von 0,2 bis 1 Gew.-% vorhanden ist; die Aminosäure in der Zusammensetzung in einer Menge von 0,2 bis 0,5 Gew.-% vorhanden ist; und das Antioxidans in der Zusammensetzung in einer Menge von 0,1 bis 0,5 Gew.-% vorhanden ist.

12. Zusammensetzung nach einem der Ansprüche 1 bis 11, ferner umfassend ein mukoadhäsives Mittel, wie z.B. eine Hyaluronsäure mit niedrigem Molekulargewicht (HA), Propylenglycolalginat (PGA), Xanthangummi, Akaziengummi, ein Carbomer (Polyacrylsäure), Chitosan, humanes Serumalbumin, Gellungummi, Guargummi, Carrageenan, und ein Cellulosederivat, wie z.B. Carboxymethylcellulose und Hydroxypropylmethylcellulose; und/oder ein Puffermittel, wie z.B. einen Puffer auf Essigsäure-, Zitronensäure-, Weinsäure-, Milchsäure-, Salzsäure- oder Hydrogenphosphorsäurebasis, Puffer auf Diethylaminbasis, Britton-Robinson-Puffer und Carmody-Puffer.

13. Zusammensetzung nach einem der Ansprüche 1 bis 12 zur Verwendung zum Fördern von (i) Regeneration von parodontalem oder orthopädischem weichem oder hartem Gewebe; (ii) Wundverschluss; oder (iii) Hautregeneration und -verjüngung.

14. Zusammensetzung zur Verwendung nach Anspruch 13, wobei das orthopädische weiche Gewebe Knorpel, Bänder oder Sehnen ist; das orthopädische harte Gewebe Knochen ist; oder das parodontale weiche oder harte Gewebe ein Gewebe ist, das aufgrund einer Parodontalerkrankung, wie z.B. Parodontitis oder Zahnfleischrezession, oder eines Parodontaltraumas, wie z.B. Oral- oder Parodontalwunde, verloren gegangen ist.

15. Zusammensetzung zur Verwendung nach Anspruch 14 zur:
(i) Behandlung von intraossären Defekten, Furkationsdefekten oder Rezessionsdefekten;
(ii) Behandlung von Gelenkknorpeldefekten, Knorpel- oder Osteochondraldefekten oder leichter bis mittelschwerer Osteoarthritis, einschließlich multipler Defekte, um Gelenkoberflächenläsionen oder Knorpelfokalläsionen zu reparieren;
(iii) Behandlung von anteriorer Kreuzbandverletzung, akuter lateraler Sprungbandverletzung vom Grad II und III oder skapholunärer interossärer Bänderverletzung / scapholunärem Bänderriss / gerissenen Handgelenkbändern; oder
(iv) Behandlung von Sehnenverletzung, lateraler Epikondylitis, Flexorsehnenverletzung, Läsionen von Fingerflexorsehnen, Sprunggelenksehnenverletzung, Sehnenverletzungen ohne Riss oder oder Tendinopathie.

16. Zusammensetzung zur Verwendung nach Anspruch 13, wobei das Fördern von Wundverschluss Unterstützung von Hautreparatur und -regeneration oder Verringern von Gewebevernarbung ist.

17. Kit, umfassend eine flüssige medizinische Zusammensetzung nach einem der Ansprüche 1 bis 12; und ein Abgabemittel, wie z.B. eine Spritze oder einen Applikator, zur Verabreichung der Zusammensetzung an eine Stelle in einem Subjekt mit Bedarf an Regeneration von parodontalem oder orthopädischem weichem oder hartem Gewebe, Wundverschluss oder Hautregeneration und - verjüngung.

## Revendications

1. Composition médicale liquide comprenant un copolymère de poloxamère thermosensible non biodégradable pharmaceutiquement acceptable en une quantité de 18 % à 30 % en poids ; de l'amélogénine en une quantité de 0,005 % à 3 % en poids ; un disaccharide en une quantité de 0,05 % à 5 % en poids ; un acide aminé choisi parmi l'alanine, la glycine, l'isoleucine, la leucine, la proline, la valine et un mélange de celles-ci en une quantité de 0,05 % à 5 % en poids ; et un antioxydant qui est un piégeur d'o-quinone choisi parmi l'acide ascorbique ou un sel de celui-ci, un dérivé d'acide ascorbique comme le 6-palmitate d'acide ascorbique, la méthionine, la N-acéthylméthionine, la cystéine, la N-acétylcystéine (NAC) et le glutathion (GSH), en une quantité de 0,01 % à 2 % en poids,
dans laquelle ladite composition a un pH de 3,8 à 5,5 et une viscosité de 50 à 2 000 millipascals secondes (mPa·s) à une température d'environ 2 à 8 °C, lorsqu'elle est mesurée avec un rhéomètre MCR 72 à une vitesse de cisaillement de 1 000 s⁻¹ ; et lorsqu'elle est chauffée à environ 37 °C, ladite composition se solidifie en un gel ayant une viscosité d'au moins 10 000 mPa s lorsqu'elle est mesurée avec un rhéomètre MCR 72 à une vitesse de cisaillement de 1 s⁻¹.

2. Composition selon la revendication 1, dans laquelle ledit copolymère de poloxamère est le poloxamère 407, le poloxamère 188, le poloxamère 124, le poloxamère 237, le poloxamère 338 ou un mélange de ceux-ci.

3. Composition selon la revendication 2, dans laquelle ledit copolymère de poloxamère est le poloxamère 407.

4. Composition selon la revendication 3, dans laquelle ledit copolymère de poloxamère est présent dans ladite composition en une quantité de 21 % à 29 %, 23 % à 28 % ou 25 % à 27 % en poids.

5. Composition selon la revendication 1, dans laquelle ladite amélogénine est une amélogénine naturelle ou une amélogénine recombinante.

6. Composition selon la revendication 5, dans laquelle ladite amélogénine est présente dans ladite composition en une quantité de 0,01 % à 1 % en poids.

7. Composition selon la revendication 1, dans laquelle :
(i) ledit disaccharide est le saccharose, le tréhalose, le lactose, le maltose, le cellobiose, le gentiobiose ou un mélange de ceux-ci ;
(ii) ledit acide aminé est la glycine ; ou
(iii) ledit antioxydant est la L-méthionine, la N-acéthylméthionine, la L-cystéine, la N-acétylcystéine (NAC), le glutathion (GSH), l'acide ascorbique, un ascorbate comme l'ascorbate de sodium, un dérivé d'acide ascorbique comme le 6-palmitate d'acide ascorbique ou un mélange de ceux-ci.

8. Composition selon la revendication 7, dans laquelle :
(i) ledit disaccharide est présent dans ladite composition en une quantité de 0,1 % à 2,5 % en poids ;
(ii) ledit acide aminé est présent dans ladite composition en une quantité de 0,1 % à 1 % en poids ; ou
(iii) ledit antioxydant est présent dans ladite composition en une quantité de 0,05 % à 1 % en poids.

9. Composition selon la revendication 1, dans laquelle ledit copolymère de poloxamère est le poloxamère 407 ; ladite amélogénine est une amélogénine recombinante ; ledit disaccharide est le tréhalose ; ledit acide aminé est la glycine ; et ledit antioxydant est la méthionine.

10. Composition selon la revendication 9, dans laquelle ledit copolymère de poloxamère est présent dans ladite composition en une quantité de 21 % à 29 % en poids ; ladite amélogénine est présente dans ladite composition en une quantité de 0,01 % à 1 % en poids ; ledit disaccharide est présent dans ladite composition en une quantité de 0,1 % à 2,5 % en poids ; ledit acide aminé est présent dans ladite composition en une quantité de 0,1 % à 1 % en poids ; ledit antioxydant est présent dans ladite composition en une quantité de 0,05 % à 1 % en poids ; et ladite composition a un pH de 4,0 à 5,0.

11. Composition selon la revendication 10, dans laquelle ledit copolymère de poloxamère est présent dans ladite composition en une quantité de 25 % à 27 % en poids ; ladite amélogénine est présente dans ladite composition en une quantité de 0,02 % à 0,5 % en poids ; ledit disaccharide est présent dans ladite composition en une quantité de 0,2 % à 1 % en poids ; ledit acide aminé est présent dans ladite composition en une quantité de 0,2 % à 0,5 % en poids ; et ledit antioxydant est présent dans ladite composition en une quantité de 0,1 % à 0,5 % en poids.

12. Composition selon l'une quelconque des revendications 1 à 11, comprenant en outre un agent muco-adhésif tel que l'acide hyaluronique de bas poids moléculaire (HA), un alginate de propylène glycol (PGA), la gomme xanthane, la gomme acacia, un carbomère (acide polyacrylique), le chitosane, la sérum albumine humaine, la gomme gellane, la gomme guar, le carraghénane et un dérivé de cellulose tel que le carboxyméthylcellulose et l'hydroxypropylméthylcellulose ; et/ou un agent tampon tel qu'un tampon à base d'acide acétique, d'acide citrique, d'acide tartrique, d'acide lactique, d'acide chlorhydrique ou d'acide hydrogénophosphorique, un tampon à base de diéthylamine, un tampon de Britton-Robinson et un tampon de Carmody.

13. Composition selon l'une quelconque des revendications 1 à 12, destinée à être utilisée pour favoriser (i) la régénération parodontale ou orthopédique de tissus mous ou durs ; (ii) la fermeture de plaies ; ou (iii) la régénération et le rajeunissement de la peau.

14. Composition destinée à être utilisée selon la revendication 13, dans laquelle ledit tissu mou orthopédique est un cartilage, des ligaments ou des tendons ; ledit tissu dur orthopédique est un os ; ou ledit tissu mou ou dur parodontal est un tissu perdu en raison d'une maladie parodontale telle que la parodontite ou une récession gingivale, ou un traumatisme parodontal tel qu'une plaie orale ou parodontale.

15. Composition destinée à être utilisée selon la revendication 14, dans :
(i) le traitement de défauts intraosseux, de défauts de furcation ou de défauts de récession ;
(ii) le traitement de défauts cartilagineux articulaires, de défauts cartilagineux ou ostéochondraux, ou l'ostéoarthrite légère à modérée comprenant des défauts multiples, réparant ainsi des lésions articulaires de surface, ou des lésions focales au cartilage ;
(iii) le traitement d'une lésion du ligament croisé antérieur, d'une lésion aiguë du ligament latéral de la cheville de grade II et III ou d'une lésion du ligament interosseux scapholuné/déchirure du ligament scapholuné/ligaments déchirés du poignet ; ou
(iv) le traitement d'une lésion tendineuse, de l'épicondylite latérale, d'une lésion du tendon fléchisseur, de lésions des tendons fléchisseurs des doigts, d'un tendon blessé de la cheville, de lésions de tendons non rompus ou d'une tendinopathie.

16. Composition destinée à être utilisée selon la revendication 13, dans laquelle ladite action favorisant la fermeture de la plaie aide à la réparation et à la régénération dermiques, ou réduit le tissu cicatriciel.

17. Kit comprenant une composition médicale liquide selon l'une quelconque des revendications 1 à 12 ; et un moyen de délivrance, tel qu'une seringue ou un applicateur, pour administrer ladite composition sur un site chez un sujet nécessitant une régénération parodontale ou orthopédique de tissus mous ou durs, une fermeture de plaie, ou une régénération et un rajeunissement de la peau.
